(19) **Europäisches Patentamt**
**European Patent Office**
**Office européen des brevets**

(11) **EP 1 409 123 B1**

(12) **EUROPEAN PATENT SPECIFICATION**

(45) Date of publication and mention
of the grant of the patent:
**04.11.2015 Bulletin 2015/45**

(21) Application number: **02740962.2**

(22) Date of filing: **17.07.2002**

(51) Int Cl.:
**B01J 2/04** *(2006.01)* **B01F 5/02** *(2006.01)*

(86) International application number:
**PCT/GB2002/003296**

(87) International publication number:
**WO 2003/008082 (30.01.2003 Gazette 2003/05)**

(54) **METHOD AND APPARATUS FOR PREPARING PARTICLES**

VERFAHREN UND VORRICHTUNG ZUR HERSTELLUNG VON TEILCHEN

PROCEDE ET APPAREIL DE PREPARATION DE PARTICULES

(84) Designated Contracting States:
**AT BE BG CH CY CZ DE DK EE ES FI FR GB GR
IE IT LI LU MC NL PT SE SK TR**

(30) Priority: **20.07.2001 GB 0117696**
**17.04.2002 GB 0208742**

(43) Date of publication of application:
**21.04.2004 Bulletin 2004/17**

(73) Proprietor: **Nektar Therapeutics
San Francisco, CA 94158 (US)**

(72) Inventors:
• **KORDIKOWSKI, Andreas
Nr Skipton BD23 4HJ (GB)**

• **PALAKODATY, Srinivas
Foster City, CA 94404 (US)**
• **GILBERT, Darren John
Bradford, BD10 8WX (GB)**

(74) Representative: **Brewster, Andrea Ruth et al
Greaves Brewster LLP
Copa House
Station Road
Cheddar, BS27 3AH (GB)**

(56) References cited:
**EP-A- 0 871 001 WO-A-00/56439
WO-A-01/66090 WO-A-97/31691
WO-A2-02/068107 DD-B- 203 688
GB-A- 203 794 GB-A- 2 187 115**

**Description**

Field of the invention

This invention relates to methods and apparatus for forming particles of a target substance.

Background to the invention

It is known to use a compressed fluid, typically a supercritical or near-critical fluid, as an anti-solvent to precipitate particles of a substance of interest (a "target substance") from solution or suspension. The basic technique is known as "GAS" (Gas Anti-Solvent) precipitation [Gallagher et al, "Supercritical Fluid Science and Technology", ACS Symp. Ser., 406, p334 (1989)]. Versions of it have been disclosed for instance in EP-0 322 687 and WO-90/03782.

In one particular version known as SEDS™ (Solution Enhanced Dispersion by Supercritical fluids), a target substance is dissolved or suspended in an appropriate fluid vehicle, and the resulting "target solution/suspension" then co-introduced into a particle formation vessel with an anti-solvent fluid (usually supercritical) in which the vehicle is soluble. The co-introduction is effected in a particular way, such that:

- the target solution/suspension and the anti-solvent both meet and enter the vessel at substantially the same point; and

- at that point, the mechanical energy of the anti-solvent serves to disperse the target solution/suspension (ie, to break it up into individual fluid elements) *at the same time as* the anti-solvent extracts the vehicle so as to cause particle formation.

Thus, in SEDS™, the compressed fluid serves not only as an anti-solvent but also as a mechanical dispersing agent. The simultaneity of fluid contact, dispersion and particle formation provides a high degree of control over the physicochemical properties of the particulate product.

Versions of SEDS™ are described in WO-95/01221, WO-96/00610, WO-98/36825, WO-99/44733, WO-99/59710, WO-01/03821, WO-01/15664 and WO-02/38127. Other SEDS™ processes are described in WO-99/52507, WO-99/52550, WO-00/30612, WO-00/30613 and WO-00/67892.

Another version of the GAS technique is described in WO-97/31691, in which a special form of two-fluid nozzle is used to introduce a "target solution/suspension" and an energising gas into a particle formation vessel containing a supercritical anti-solvent. The energising gas can be the same as the anti-solvent fluid. Within the nozzle, a restriction generates sonic waves in the energising gas/anti-solvent flow and focusses them back (ie, in a direction opposite to that of the energising gas flow) on the outlet of the target solution/suspension passage, resulting in mixing of the fluids within the nozzle before they enter the particle formation vessel. It is suggested that where the energising gas is the same as the anti-solvent (typically supercritical carbon dioxide), its flow rate could be sufficiently high to obtain a sonic velocity at the nozzle outlet. However, the authors do not appear ever to have achieved such high velocities in their experimental examples.

Other modifications have been made to the basic GAS process in order to affect atomisation of the target solution/suspension at the point of its contact with the compressed fluid anti-solvent. For example, US-5,770,559 describes a GAS precipitation process in which a target solution is introduced, using a sonicated spray nozzle, into a pressure vessel containing a supercritical or near-critical anti-solvent fluid - see also Randolph et al in Biotechnol. Prog., 1993, 9, 429-435.

WO-00/56439 describes a GAS particle formation method and apparatus, in which a first supercritical fluid is co-introduced into a particle formation vessel with a solution or suspension of a target material, and a second supercritical fluid is introduced separately into the vessel at an outlet distant from the point of entry of the first supercritical fluid.

GB-2 187 115 describes a method for producing a spray powder in which a starting slurry or liquid is sprayed simultaneously through a plurality of spaced apart nozzles, the nozzles producing overlapping, substantially conical, spray patterns.

WO-97/31691 describes a method and apparatus for particle precipitation and coating, in which a supercritical or near-critical fluid anti-solvent is introduced into a particle formation vessel at a location separate to the point of introduction of a dispersion containing a precipitable substance.

WO-01/66090 describes another GAS particle formation system, in which again a supercritical fluid anti-solvent is introduced into a particle formation vessel separately to a solution of a substance to be precipitated.

It would be generally desirable to provide alternative particle formation techniques which combined one or more of the advantages of the prior art methods with a broader applicability (for instance, for a wider range of target substances; vehicles and/or anti-solvents) and/or a higher degree of control over the product characteristics. In particular it is generally desirable, especially for pharmaceutical substances, to be able to produce small (even sub-micron) particles with narrow size distributions.

Statements of the invention

According to a first aspect of the present invention there is provided a method for preparing a target substance in particulate form, the method comprising introducing into a particle formation vessel, through separate first and second fluid inlet means respectively, (a) a solution or suspension of the target substance in a fluid vehicle (the "target solution/suspension") and (b) a compressed fluid anti-solvent for the substance, and allowing the anti-solvent fluid to extract the vehicle from the target solution/suspension so as to form particles of the target substance, wherein the anti-solvent fluid has a sonic, near-sonic or supersonic velocity as it enters the particle formation vessel, and wherein the anti-solvent and the target solution/suspension enter the particle formation vessel at different locations and meet downstream (in the direction of anti-solvent flow) of the second fluid inlet means and the pressure drop as the anti-solvent fluid enters the particle formation vessel is from 170 to 250 bar.

By "sonic velocity" and "supersonic velocity" is meant respectively that the velocity of the anti-solvent fluid as it enters the vessel is the same as or greater than the velocity of sound in that fluid at that point. By "near-sonic velocity" is meant that the anti-solvent velocity on entry into the vessel is slightly lower than, but close to, the velocity of sound in that fluid at that point - for instance its "Mach number" M (the ratio of its actual speed to the speed of sound) is greater than 0.8, preferably greater than 0.9 or 0.95. Generally speaking, in the method of the invention, the Mach number for the anti-solvent fluid on entering the particle formation vessel may be between 0.8 and 1.5, preferably between 0.9 and 1.3.

A near-sonic, sonic or supersonic anti-solvent velocity may be achieved by selecting appropriate operating conditions, in particular the temperature and pressure of the fluid as it enters the particle formation vessel, the temperature and pressure within the vessel (which may be controlled in conventional manner, for instance using an oven and a back pressure regulator) and the geometry (in particular size) of the inlet through which the anti-solvent is introduced into the vessel.

References in this specification to a fluid entering a vessel are to the fluid exiting an inlet means (for example, a nozzle) used to introduce the fluid into the vessel. For these purposes, therefore, the inlet means is to be considered as *upstream of* the vessel in the direction of fluid flow, although parts of it (in particular its outlet) may be located physically within the vessel.

There needs to be a drop in pressure as the anti-solvent fluid enters the particle formation vessel. This is typically achieved by imparting a relatively high "back pressure" to the anti-solvent (by using a high anti-solvent flow rate and forcing it through a restriction such as a nozzle) and maintaining the vessel at a significantly lower pressure.

However, this pressure reduction can cause undesirable Joule-Thomson cooling of the anti-solvent. Accordingly, the temperature of the anti-solvent upstream of the particle formation vessel needs to be sufficiently high that the fluid remains at an appropriate temperature (typically above its critical temperature $T_c$), even after expanding into the particle formation vessel. The method of the invention thus preferably includes preheating the anti-solvent fluid, upstream of the particle formation vessel, to a temperature such as to compensate for its Joule-Thomson cooling as it enters the vessel.

Thus, the first aspect of the present invention may be seen as a method for preparing a target substance in particulate form, the method comprising introducing into a particle formation vessel (a) a solution or suspension of the target substance in a fluid vehicle (the "target solution/suspension") and (b) a compressed fluid anti-solvent for the substance, and allowing the anti-solvent fluid to extract the vehicle from the target solution/suspension so as to form particles of the target substance, wherein (i) the pressure in the particle formation vessel is $P_1$ which is preferably greater than the critical pressure $P_c$ of the anti-solvent, (ii) the anti-solvent is introduced through a restricted inlet so as to have a back pressure of $P_2$, where $P_2$ is greater than $P_1$, (iii) the temperature in the particle formation vessel is $T_1$ which is preferably greater than the critical temperature $T_c$ of the anti-solvent, (iv) the anti-solvent is introduced into the vessel at a temperature $T_2$, where $T_2$ is greater than $T_1$, (v) $T_1$ and $T_2$ are such that Joule-Thomson cooling of the anti-solvent as it enters the vessel does not reduce the anti-solvent temperature to below that required of it at the point of particle formation (and are preferably such that the anti-solvent temperature does not fall below $T_c$ within the vessel) and (vi) $P_1$, $P_2$, $T_1$ and $T_2$ are such that the anti-solvent fluid has a sonic, near-sonic or supersonic velocity as it enters the particle formation vessel.

Again the anti-solvent and the target solution/suspension must be introduced separately into the particle formation vessel and contact each other downstream of (preferably immediately downstream of) the point of anti-solvent entry into the vessel.

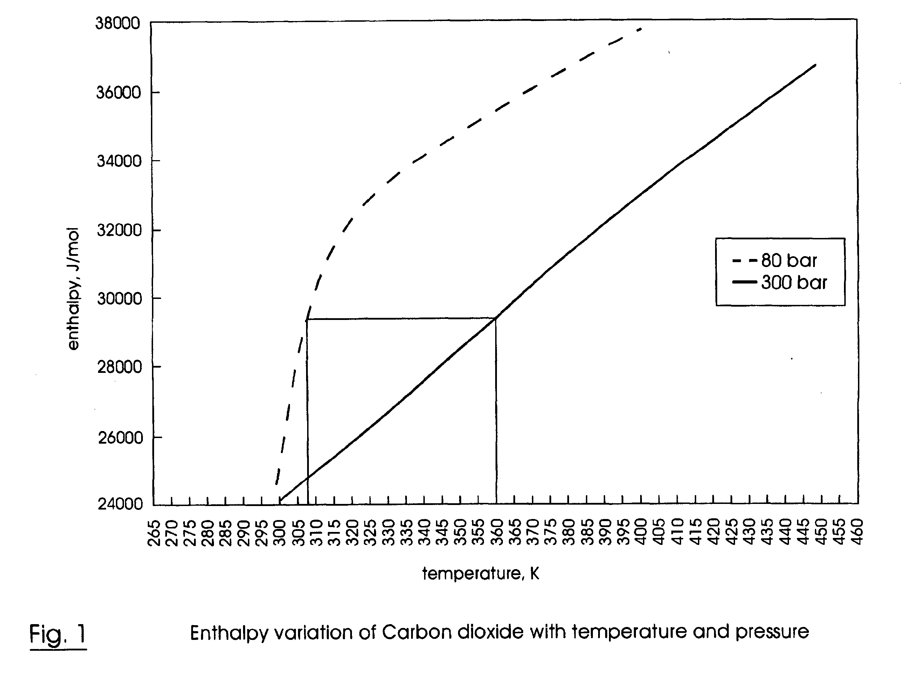

The anti-solvent expansion as it enters the particle formation vessel is isenthalpic. Thus, an appropriate temperature for the anti-solvent upstream of the vessel may be derived from enthalpy charts for the fluid, for instance as illustrated for carbon dioxide in Fig 1. (For $CO_2$, the critical temperature $T_c$ is 31 °C (304 K) and the critical pressure $P_c$ is 74 bar.) Fig 1 shows how, when working with a pressure reduction from 300 to 80 bar for the $CO_2$ on entering the particle formation vessel, the upstream temperature should be at least 360 K (87 °C) to achieve an appropriate temperature of 308 K (35 °C) or greater when the $CO_2$ enters the vessel.

Thus, a carbon dioxide anti-solvent is preferably introduced with an upstream temperature of 80 °C (353 K) or higher, more preferably between 80 °C and 170 °C (443 K).

The pressures and temperatures needed to ensure a near-sonic, sonic or supersonic velocity depend on the nature of the anti-solvent fluid. In the case of a carbon dioxide anti-solvent, for instance, in order to achieve a sonic or supersonic velocity the operating conditions must satisfy the formula:

$$\frac{p_o}{p_i} \le \left[ \frac{2}{k+1} \right]^{\frac{k}{k-1}}$$

where $p_i$ is the $CO_2$ pressure upstream of entry into the particle formation vessel and $p_o$ is the $CO_2$ pressure immediately on entry into the vessel, and $k$ is the ratio of the specific heats of $CO_2$ at constant pressure ($C_p$) and constant volume ($C_v$).

So, for example the $CO_2$ may be introduced at a temperature of 360 K (87 °C) with an inlet pressure $p_i$ of 300 bar, and the vessel may be at 310 K (37 °C) and 80 bar (ie, the outlet pressure $p_o$ is 80 bar). At 310 K and 80 bar, $k$ for $CO_2$ is 8.78[1]. At 360 K and 300 bar, $k$ is 2.29[1]. Taking a geometric average for $k$ of 4.48, as the $CO_2$ exits the nozzle, then substituting these values into the above equation gives $\dfrac{p_o}{p_i} = 0.267$ and $\left[ \dfrac{2}{k+1} \right]^{\frac{k}{k-1}} = 0.274$, which confirms that the $CO_2$ flow is supersonic irrespective of the $CO_2$ flow rate into the vessel, so long as there is an appropriate pressure differential between $p_i$ and $p_o$. A suitable $CO_2$ flow might be for instance between 170 and 200 g/min. The pressure drop as the $CO_2$ enters the particle formation vessel is between 170 and 250 bar.

An alternative method for calculating the anti-solvent velocity (again for carbon dioxide, using the same operating conditions as above but with a vessel temperature of 40 °C, and introducing the $CO_2$ through a nozzle of outlet diameter 0.2 mm) is:

> (i) density of $CO_2$ at 310 K and 80 bar[1] is 0.33088 g/cm$^3$,
> (ii) therefore, volumetric flow of $CO_2$ at 200 g/min (Q) is 200/0.33088 = 604.45 cm$^3$/min.
> (iii) Surface area (A) of the nozzle = 3.14 x 10$^{-4}$ cm$^2$,
> (iv) therefore velocity of $CO_2$ = Q ÷ (A x 60 x 100) = 320.7 m/s.
> (v) Speed of sound in $CO_2$ at 310 K and 80 bar[1] is 196.8 m/s.
> (vi) Thus, the $CO_2$ velocity is confirmed as being supersonic under such conditions.

Although we do not wish to be bound by this theory, it is believed that in the method of the invention, a so-called "Mach disk" is generated in the anti-solvent flow downstream [1]International thermodynamic tables of the fluid state - 3. Carbon dioxide, Angus et al, Pergamon Press, 1976 of the second fluid inlet means. In this region the fluid velocity will change abruptly to sub-sonic thus generating shock waves in the fluids present (in effect a continuous, low volume, supersonic boom). These shock waves are thought to aid mixing and dispersion of the target solution/suspension with the anti-solvent. It is unlikely that the waves will be ultrasonic as in for example the system described in WO-97/31691. Moreover they will propagate in the direction of the anti-solvent flow, rather than in a counter-current sense as in for instance the nozzle described in WO-97/31691 which reflects a sonic wave back towards a source of energising gas.

The arrangement of the first and second inlet means will preferably be such that the Mach disk is generated upstream (in the direction of anti-solvent flow) of the point of entry of the target solution/suspension into the particle formation vessel. It should occur in line with the longitudinal axis of the second inlet means, ie, in line with the direction of anti-solvent flow.

The near-sonic, sonic or supersonic anti-solvent velocity is ideally achieved, in the method of the present invention, simply by the use of appropriate anti-solvent flow rates, back pressures and/or operating temperatures, and without the aid of mechanical, electrical and/or magnetic input such as for example from impellers, impinging surfaces especially within the anti-solvent introducing means, electrical transducers and the like. Introducing the anti-solvent via a convergent nozzle, ideally as a single fluid stream, may also help in the achievement of appropriate fluid velocities. Further "energising" fluid streams, such as those required in the method of WO-97/31691, are not then needed in order to achieve the desired level of control over the contact between the target solution/suspension and the anti-solvent fluid.

The use of near-sonic, sonic or supersonic anti-solvent velocities can allow achievement of smaller particle sizes and narrower size distributions in GAS-based particle formation processes. In particular it can allow the formation of small micro- or even nanoparticles, for instance of volume mean diameter less than 5 $\mu$m, preferably less than 2 $\mu$m, more preferably less than 1 $\mu$m. Such particulate products preferably have narrow size distributions, such as with a standard deviation of 2.5 or less, more preferably 2.0 or less, most preferably 1.9 or even 1.8 or less.

The use of near-sonic, sonic or supersonic anti-solvent velocities also appears to lead to more efficient vehicle extraction, thus potentially yielding particles with lower residual solvent levels, less agglomeration and generally improved handling properties.

The anti-solvent fluid must be in a compressed state, by which is meant that it is above its vapour pressure, preferably above atmospheric pressure, more preferably from 70 to 200 bar or from 80 to 150 bar. More preferably "compressed" means above the critical pressure $P_c$ for the fluid or fluid mixture concerned. In practice, the pressure of the anti-solvent fluid is likely to be in the range $(1.01 - 9.0)P_c$, preferably $(1.01 - 7.0)P_c$.

Thus, the anti-solvent is preferably a supercritical or near-critical fluid, although it may alternatively be a compressed liquid such as for instance liquid $CO_2$.

As used herein, the term "supercritical fluid" means a fluid at or above its critical pressure ($P_c$) and critical temperature ($T_c$) simultaneously. In practice, the pressure of the fluid is likely to be in the range $(1.01 - 9.0)P_c$, preferably $(1.01 - 7.0)P_c$, and its temperature in the range $(1.01 - 4.0)T_c$ (measured in Kelvin). However, some fluids (eg, helium and neon) have particularly low critical pressures and temperatures, and may need to be used under operating conditions well in excess of (such as up to 200 times) those critical values.

"Near-critical fluid" is here used to refer to a fluid which is either (a) above its $T_c$ but slightly below its $P_c$, (b) above its $P_c$ but slightly below its $T_c$ or (c) slightly below both its $T_c$ and its $P_c$. The term "near-critical fluid" thus encompasses both high pressure liquids, which are fluids at or above their critical pressure but below (although preferably close to) their critical temperature, and dense vapours, which are fluids at or above their critical temperature but below (although preferably close to) their critical pressure.

By way of example, a high pressure liquid might have a pressure between about 1.01 and 9 times its $P_c$, and a temperature between about 0.5 and 0.99 times its $T_c$. A dense vapour might, correspondingly, have a pressure between about 0.5 and 0.99 times its $P_c$, and a temperature between about 1.01 and 4 times its $T_c$.

The terms "supercritical fluid" and "near-critical fluid" each encompass a mixture of fluid types, so long as the mixture is in the supercritical or near-critical state respectively.

In the method of the present invention, it may be preferred that the operating temperature (ie, the temperature in the particle formation vessel) be close to the critical temperature $T_c$ of the mixture of anti-solvent and target solution/suspension formed at the point of fluid contact. For example, the temperature might be between 0.9 and 1.1 times $T_c$ (in Kelvin), preferably between 0.95 and 1.05 times $T_c$, more preferably between 0.97 and 1.03 or between 0.98 and 1.02 times $T_c$, or perhaps between 1 and 1.05 or 1 and 1.03 or 1 and 1.02 times $T_c$. This is because at $T_c$ the velocity of sound in a fluid is theoretically zero; near-sonic, sonic and supersonic velocities can thus more readily be achieved, using lower anti-solvent flow rates, as $T_c$ is approached.

The anti-solvent should be a compressed (preferably supercritical or near-critical, more preferably supercritical) fluid at its point of entry into the particle formation vessel and preferably also within the vessel and throughout the particle formation process. Thus, for a carbon dioxide anti-solvent the temperature in the particle formation vessel is ideally greater than 31 °C, for example between 31 and 100 °C, preferably between 31 and 70°C, and the pressure greater than 74 bar, for example between 75 and 350 bar.

Carbon dioxide is a highly suitable anti-solvent, but others include nitrogen, nitrous oxide, sulphur hexafluoride, xenon, ethylene, chlorotrifluoromethane, ethane, trifluoromethane and noble gases such as helium or neon.

The anti-solvent must be miscible or substantially miscible with the fluid vehicle at the point of their contact, so that the anti-solvent can extract the vehicle from the target solution/suspension. By "miscible" is meant that the two fluids are miscible in all proportions, and "substantially miscible" encompasses the situation where the fluids can mix sufficiently well, under the operating conditions used, as to achieve the same or a similar effect, ie, dissolution of the fluids in one another and precipitation of the target substance. However the anti-solvent must not, at the point of particle formation, extract or dissolve the target substance. In other words, it must be chosen so that the target substance is for all practical purposes (in particular, under the chosen operating conditions and taking into account any fluid modifiers present) insoluble or substantially insoluble in it. Preferably the target substance is less than $10^{-3}$ mole %, more preferably less than $10^{-5}$ mole %, soluble in the anti-solvent fluid.

The anti-solvent fluid may optionally contain one or more modifiers, for example water, methanol, ethanol, isopropanol or acetone. A modifier (or co-solvent) may be described as a chemical which, when added to a fluid such as a supercritical or near-critical fluid, changes the intrinsic properties of that fluid in or around its critical point, in particular its ability to dissolve other materials. When used, a modifier preferably constitutes not more than 40 mole %, more preferably not more than 20 mole %, and most preferably between 1 and 10 mole %, of the anti-solvent fluid.

The vehicle is a fluid which is able to carry the target substance in solution or suspension. It may be composed of one or more component fluids, eg, it may be a mixture of two or more solvents. It must be soluble (or substantially soluble) in the chosen anti-solvent fluid at their point of contact. It may contain, in solution or suspension, other materials apart from the target substance.

The target solution/suspension may in particular comprise two or more fluids which are mixed *in situ* at or immediately before their contact with the anti-solvent. Such systems are described, eg, in WO-96/00610 and WO-01/03821. The two or more fluids may carry two or more target substances, to be combined in some way (for instance, coprecipitated as a matrix, or one precipitated as a coating around the other, or precipitated as the product of an *in situ* reaction between the substances) at the point of particle formation. Target substance(s) may also be carried in the anti-solvent fluid as well as in the target solution(s)/suspension(s).

The target substance may be any substance which needs to be produced in particulate form. Examples include pharmaceuticals; pharmaceutical excipients such as carriers; dyestuffs; cosmetics; foodstuffs; coatings; agrochemicals; products of use in the ceramics, explosives or photographic industries; etc... It may be organic or inorganic, monomeric or polymeric. It is preferably soluble or substantially soluble in the fluid vehicle, preferably having a solubility in it of $10^{-4}$ mole % or greater under the conditions under which the target solution is prepared (ie, upstream of the point of particle formation).

In a preferred embodiment of the invention, the target substance is for use in or as a pharmaceutical or pharmaceutical excipient.

The target substance may be in a single or multi-component form (eg, it could comprise an intimate mixture of two materials, or one material in a matrix of another, or one material coated onto a substrate of another, or other similar mixtures). The particulate product, formed from the target substance using the method of the invention, may also be in such a multi-component form - examples include two pharmaceuticals intended for co-administration, or a pharmaceutical together with a polymer carrier matrix. Such products may be made (as described above) from solutions/suspensions containing only single component starting materials, provided the solutions/suspensions are contacted with the anti-solvent fluid in the correct manner. The particulate product may comprise a substance formed from an *in situ* reaction (ie, immediately prior to, or on, contact with the anti-solvent) between two or more reactant substances each carried by an appropriate vehicle.

In the method of the invention, the anti-solvent and the target solution/suspension are introduced separately into the particle formation vessel (which is preferably the vessel in which the formed particles are collected) and contact each other after (preferably immediately after) their point of entry into the vessel. In this way, particle formation can be made to occur at a point where there is a high degree of control over conditions such as the temperatures, pressures and flow rates of the fluids.

The fluids are ideally introduced in such a way that the mechanical (kinetic) energy of the anti-solvent fluid can act to disperse the target solution/suspension at the same time as it extracts the vehicle; this again allows a high degree of control over the physicochemical characteristics of the particulate product, in particular the size and size distribution of the particles and their solid state properties. "Disperse" in this context refers generally to the transfer of kinetic energy from one fluid to another, usually implying the formation of droplets, or of other analogous fluid elements, of the fluid to which the kinetic energy is transferred. Thus, the fluid inlet means used to introduce the fluids should allow the mechanical energy (typically the shearing action) of the anti-solvent flow to facilitate intimate mixing of the fluids and to disperse them, at the point where the fluids meet.

Introducing the two fluids separately in this way can help prevent apparatus blockages at the point of anti-solvent entry, due for example to the highly efficient extraction of the vehicle into the anti-solvent under the operating conditions used.

Thus, the present invention may be seen as a modification of the SEDS™ process, in which the target solution/suspension and the anti-solvent fluid contact one another externally of their respective (preferably separate) fluid inlets into the particle formation vessel. A high degree of control is retained over the mechanism for fluid contact, as in the basic SEDS™ process, and this control may be achieved for example at least partly by introducing the anti-solvent fluid with a sonic, near-sonic or supersonic velocity. Other ways in which such control may be achieved or improved upon include providing controlled agitation within the particle formation vessel, in particular in the region of fluid contact immediately downstream of the respective target solution/suspension and anti-solvent inlets. For example, the target solution/suspension may be dispersed onto a sonicating surface at or immediately prior to its contact with the anti-solvent fluid. Agitation may alternatively be achieved for instance by stirring, such as with a turbine, propeller, paddle, impeller or the like.

That said, the present invention may if necessary be practised in the absence of such additional agitation means within the particle formation vessel.

The target solution/suspension may be introduced into the vessel through any suitable fluid inlet means, including one which effects, or assists in effecting, controlled atomisation of the solution/suspension.

Preferably the two fluids meet immediately downstream of the point of anti-solvent entry. "Immediately" in this context implies a sufficiently small time interval (between the anti-solvent entering the particle formation vessel and its contact with the target solution/suspension) as preferably still to allow transfer of mechanical energy from the anti-solvent to the solution/suspension so as to achieve dispersion. Nevertheless, there is still preferably a short interval of time between anti-solvent entry and fluid contact so as to eliminate, or substantially eliminate or at least reduce, the risk of apparatus blockage due to particle formation at the point of anti-solvent entry. The timing of the fluid contact will depend on the natures of the fluids, the target substance and the desired end product, as well as on the size and geometry of the particle formation vessel and the apparatus used to introduce the fluids and on the fluid flow rates. The contact may occur within 0.5 to 10 seconds, more preferably within 1 to 7 seconds, most preferably within 1.2 to 6 seconds, such as within 1.4 to 5.5 seconds, of the anti-solvent entering the particle formation vessel.

The target solution/suspension is preferably introduced directly into the anti-solvent flow. It preferably meets with the anti-solvent flow at the point where the target solution/suspension enters the vessel.

Preferably the outlet of the first fluid inlet means is located vertically below that of the second fluid inlet means, and the anti-solvent fluid flows into the particle formation vessel in a vertically downwards direction.

At the point where the target solution/suspension and the anti-solvent meet, the angle between their axes of flow may be from 0 ° (ie, the two fluids are flowing in parallel directions) to 180 ° (ie, oppositely-directed flows). However, they preferably meet at a point where they are flowing in approximately perpendicular directions, ie, the angle between their axes of flow is from 70 to 110 °, more preferably from 80 to 100 °, such as 90 °.

Suitable fluid inlet means, which may be used to achieve the form of fluid contact required by the first aspect of the invention, is described below in connection with the second aspect.

Use of such a fluid inlet system can allow SEDS™ and other GAS-based particle formation techniques to be practised in cases where the vehicle for the target solution/suspension is a relatively high boiling fluid (eg, boiling point greater than about 150 °C, or even greater than 180 °C) such as dimethyl formamide (DMF), dimethyl sulphoxide (DMSO), dimethyl acetamide (DMA), diethyl acetamide (DEA) or N-methyl pyrollidinone (NMP), or where the target substance is temperature sensitive. Since the anti-solvent and the target solution/suspension enter the vessel separately, the latter can be maintained at a desired lower temperature despite the use of a relatively high temperature for the incoming anti-solvent. Moreover, the use of a sonic, near-sonic or supersonic anti-solvent velocity can be sufficient to disperse the target solution/suspension at relatively low operating temperatures (ie, vessel temperatures) - again this assists in the processing of temperature sensitive target substances and vehicles.

When carrying out the present invention, the particle formation vessel temperature and pressure are ideally controlled so as to allow particle formation to occur at or substantially at the point where the target solution/suspension meets the anti-solvent fluid. The conditions in the vessel must generally be such that the anti-solvent fluid, and the solution which is formed when it extracts the vehicle, both remain in the compressed (preferably supercritical or near-critical, more preferably supercritical) form whilst in the vessel. For the supercritical, near-critical or compressed solution, this means that at least one of its constituent fluids (usually the anti-solvent fluid, which in general will be the major constituent of the mixture) should be in a compressed state at the time of particle formation. There should at that time be a *single-phase* mixture of the vehicle and the anti-solvent fluid, otherwise the particulate product might be distributed between two or more fluid phases, in some of which it might be able to redissolve. This is why the anti-solvent fluid needs to be miscible or substantially miscible with the vehicle.

The terms "supercritical solution", "near-critical solution" and "compressed solution" mean respectively a supercritical, near-critical or compressed fluid together with a fluid vehicle which it has extracted and dissolved. The solution should itself still be in the supercritical, near-critical or compressed state, as the case may be, and exist as a single phase, at least within the particle formation vessel.

Selection of appropriate operating conditions will be influenced by the natures of the fluids involved (in particular, their $P_c$ and $T_c$ values and their solubility and miscibility characteristics) and also by the characteristics desired of the particulate end product, for instance yield, particle size and size distribution, purity, morphology, or crystalline, polymorphic or isomeric form. Variables include the flow rates of the anti-solvent fluid and the target solution/suspension, the concentration of the target substance in the vehicle, the temperature and pressure inside the particle formation vessel, the anti-solvent temperature upstream of the vessel and the geometry of the fluid inlets into the vessel, in particular the size of the anti-solvent inlet. The method of the invention preferably involves controlling one or more of these variables so as to influence the physicochemical characteristics of the particles formed.

The flow rate of the anti-solvent fluid relative to that of the target solution/suspension, and its pressure and temperature, should be sufficient to allow it to accommodate the vehicle, so that it can extract the vehicle and hence cause particle formation. The anti-solvent flow rate will generally be higher than that of the target solution/suspension - typically, the ratio of the target solution/suspension flow rate to the anti-solvent flow rate (both measured at or immediately prior to the two fluids coming into contact with one another) will be 0.001 or greater, preferably from 0.01 to 0.2, more preferably from about 0.03 to 0.1.

The anti-solvent flow rate will also generally be chosen to ensure an excess of the anti-solvent over the vehicle when the fluids come into contact, to minimise the risk of the vehicle re-dissolving and/or agglomerating the particles formed. At the point of extraction of the vehicle it may constitute from 1 to 80 mole %, preferably 50 mole % or less or 30 mole % or less, more preferably from 1 to 20 mole % and most preferably from 1 to 5 mole %, of the compressed fluid mixture formed.

Both the anti-solvent and the target solution/suspension are ideally introduced into the particle formation vessel with a smooth, continuous and preferably pulse-less or substantially pulse-less flow. Conventional apparatus may be used to ensure such fluid flows.

The method of the invention preferably additionally involves collecting the particles following their formation, more preferably in the particle formation vessel itself

According to a second aspect of the present invention, there is provided apparatus for use in preparing a target substance in particulate form, and in particular for use in a method according to the first aspect of the invention, the apparatus comprising:

(i) a particle formation vessel;

(ii) first fluid inlet means for introducing into the vessel a solution or suspension of the target substance in a fluid vehicle (the "target solution/suspension"); and

(iii) second fluid inlet means, separate from the first, for introducing a compressed fluid anti-solvent into the particle formation vessel;

wherein the first and second fluid inlet means are so arranged that, in use, a target solution/suspension introduced through the first and an anti-solvent introduced through the second enter the particle formation vessel at different locations and meet immediately downstream (in the direction of anti-solvent flow) of the second fluid inlet means.

The first fluid inlet means suitably comprises a fluid inlet tube, for instance of stainless steel, which might typically have an internal diameter of from 0.1 to 0.2 mm, more preferably from 0.1 to 0.15 mm, and may have a tapered outlet section.

The second fluid inlet means preferably provides a restriction at the point of fluid entry into the particle formation vessel: for instance, the second fluid inlet means may comprise a nozzle. Again it may suitably be made from stainless steel. It preferably has at least one passage of internal diameter from for instance 1 to 2 mm, more preferably from 1.3 to 1.9 mm, such as 1.6 mm. Again, it may have a tapered outlet section (ie, be a "convergent"-type nozzle), with an angle of taper (with respect to the longitudinal axis of the nozzle) typically in the range 10 ° to 60 °, preferably from 10 ° to 50 °, more preferably from 20 ° to 40 °, and most preferably about 30 °.

The opening at the outlet end (tip) of the nozzle will preferably have a diameter in the range of 0.005 to 5 mm, more preferably 0.05 to 2 mm, most preferably from 0.1 to 0.5 mm, for instance about 0.1, 0.2, 0.3 or 0.35 mm.

The dimensions of the fluid inlet will naturally depend on the scale on which the process is to be practised; for commercial scale manufacture, for example, the above nozzle dimensions may be up to ten times larger.

A nozzle of the above type may comprise more than one fluid passage; for instance it may comprise two or more coaxial passages such as in the nozzles described in WO-95/01221, WO-96/00610 and WO-98/36825, particularly if additional fluids are to be introduced into the system. One or more of the passages may be used to introduce two or more fluids at the same time, and the inlets to such passages may be modified accordingly.

The outlet of the first fluid inlet means (into the particle formation vessel) is preferably immediately downstream, in the direction of anti-solvent flow in use, of that of the second fluid inlet means. A suitable separation for the two outlets is a short distance such as from 0 to 50, preferably from 10 to 40, for instance about 20 times the diameter of the outlet of the second fluid inlet means. Suitable distances might lie from 0 to 10 mm or from 0.1 to 10 mm, preferably from 2 to 8 mm, for instance about 4 mm. Again, they may depend on the scale of the process which the inlet means are to be used for.

The outlet of the first fluid inlet means preferably has a smaller cross sectional area than that of the second fluid inlet means, more preferably less than 80 % as large and most preferably less than 70 % or 65 % as large. Preferably this outlet is positioned such that, in use, it is within the flow of anti-solvent fluid exiting the second fluid inlet means. Most preferred is an arrangement in which the centre of the outlet of the first fluid inlet means corresponds to the centre of the outlet of the second fluid inlet means, ie, the centres of the two outlets are both positioned on the central longitudinal axis of the second fluid inlet means.

The first and second fluid inlet means are preferably arranged so that at the point where the target solution/suspension and the anti-solvent meet, the angle between their axes of flow is from 70 ° to 110 °, more preferably from 80 to 100 °, most preferably about 90 °.

The first and second fluid inlet means may for convenience be provided as part of a single fluid inlet assembly which may be placed in fluid communication with the particle formation vessel and with sources of the anti-solvent fluid and the target solution/suspension.

Thus, according to a third aspect, the present invention provides a fluid inlet assembly for use as part of apparatus according to the second aspect of the invention, and/or in a method according to the first aspect.

In apparatus according to the second aspect of the invention, the particle formation vessel preferably contains particle collection means, such as a filter, by which particles of the target substance may be collected in the vessel in which they form, downstream of the point of contact between the target solution/suspension and the anti-solvent fluid.

The apparatus may additionally comprise a source of a compressed (preferably supercritical or near-critical) fluid and/or a source of a target solution or suspension. The former may itself comprise means for altering the temperature and/or pressure of a fluid so as to bring it into a compressed (preferably supercritical or near-critical) state. The apparatus conveniently includes means for controlling the pressure in the particle formation vessel, for example a back pressure regulator downstream of the vessel, and/or means (such as an oven) for controlling the temperature in the vessel. The vessel is conveniently a pressure vessel and should be capable of withstanding the pressures necessary to maintain compressed (preferably supercritical or near-critical) conditions during the particle formation process, as described above in connection with the method of the invention.

Because embodiments of the present invention are modified versions of the inventions disclosed in WO-95/01221, WO-96/00610, WO-98/36825, WO-99/44733, WO-99/59710, WO-01/03821, WO-01/15664 and WO-02/38127, technical features described in those documents, for instance regarding the selection of appropriate reagents and operating conditions, can apply also to the present invention. The eight earlier documents are therefore intended to be read together with the present application.

In this specification the term "substantially", when applied to a condition, is meant to encompass the exact condition (eg, exact simultaneity) as well as conditions which are (for practical purposes, taking into account the degree of precision with which such conditions can be measured and achieved) close to that exact condition, and/or which are similar enough to that exact condition as to achieve, in context, the same or a very similar effect.

References to solubilities and miscibilities, unless otherwise stated, are to the relevant fluid characteristics under the operating conditions used, ie, under the chosen conditions of temperature and pressure and taking into account any modifiers present in the fluids.

The present invention will now be illustrated with reference to the following nonlimiting examples and the accompanying figures, of which:

Fig 1 is a plot of the enthalpy variation of $CO_2$ with temperature and pressure, illustrating the change in $CO_2$ temperature during its isenthalpic expansion;

Fig 2 illustrates schematically apparatus suitable for use in carrying out a method according to the present invention;

Figs 3 to 5 are schematic longitudinal cross sections and an under plan view respectively of parts of a fluid inlet assembly useable with the Fig 2 apparatus;

Figs 6 to 9 are SEM (scanning electron microscope) photographs of the products of Examples A1, A2, A5 and A6 (below) respectively;

Fig 10 to 12 show particle size distributions for the products of Examples B1 to B3 respectively;

Figs 13 and 14 are SEM photographs of the products of Examples D1 and D2 respectively;

Figs 15 and 16 show particle size distributions for the products of Examples D1 and D2 respectively; and

Figs 17 and 18 are SEM photographs of the products of Examples E2 and E3 respectively.

Detailed description

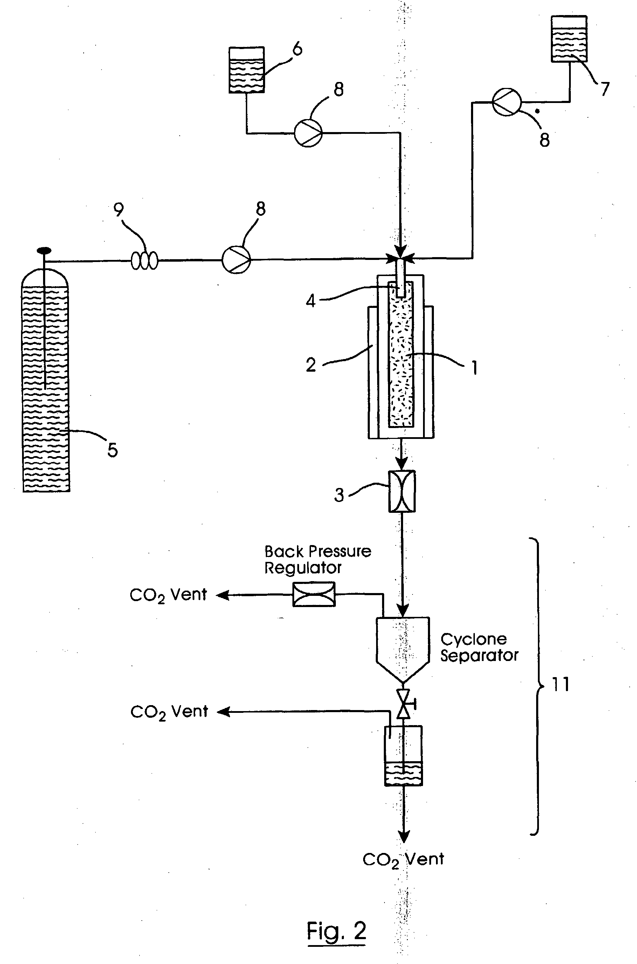

Fig 2 shows apparatus suitable for carrying out methods in accordance with the present invention. Item 1 is a particle formation vessel, within which the temperature and pressure can be controlled by means of the heating jacket 2 and back pressure regulator 3. The vessel 1 contains a particle collection device (not shown) such as a filter, filter basket or filter bag. A fluid inlet assembly 4 allows introduction of a compressed (typically supercritical or near-critical) fluid anti-solvent from source 5 and one or more target solutions/suspensions from sources such as 6 and 7. The items labelled 8 are pumps, and 9 is a cooler. A recycling system 11 allows solvent recovery.

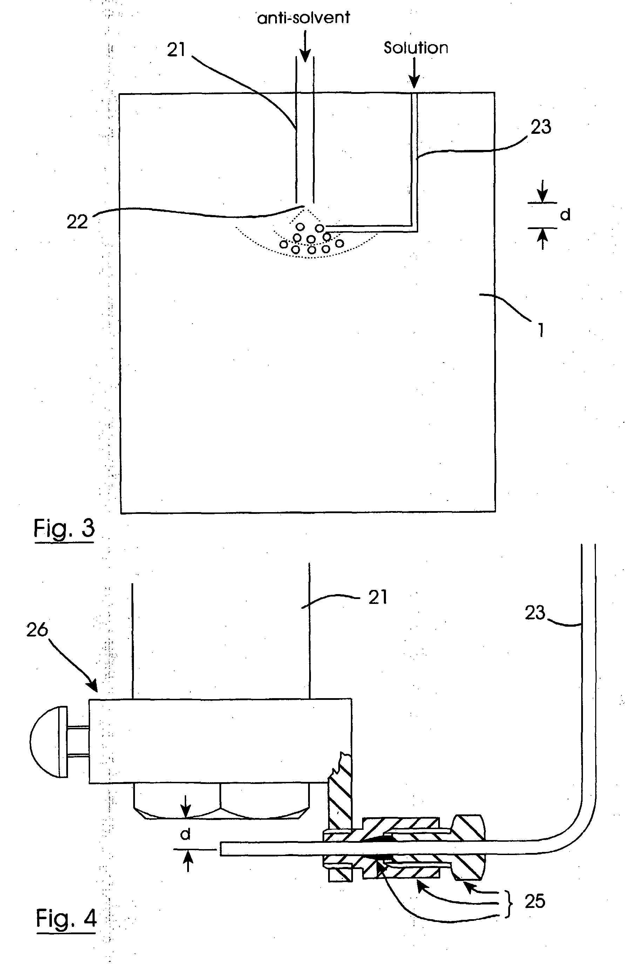

The fluid inlet assembly 4 may for example take the form shown in Figs 3 to 5. Fig 3 shows the assembly schematically, in use with the particle formation vessel 1 of the Fig 2 apparatus. Nozzle 21 is for introduction of the anti-solvent fluid. It has only a single passage of circular cross section, with a circular outlet 22. Alternatively, a multi-component nozzle may be used, with anti-solvent introduced through one or more of its passages and the remaining passages either closed off or else used to introduce additional reagents. (For example, a multi-passage nozzle of the type described in WO-95/01221 or WO-96/00610 may be used. Such nozzles have two or more concentric (coaxial) passages, the outlets of which are typically separated by a short distance to allow a small degree of internal mixing to take place between fluids introduced through the respective passages before they exit the nozzle. The anti-solvent could for instance be introduced through the inner passage of such a nozzle, traversing a small "mixing" zone as it exits that inner passage and then passing through the main nozzle outlet into the particle formation vessel.)

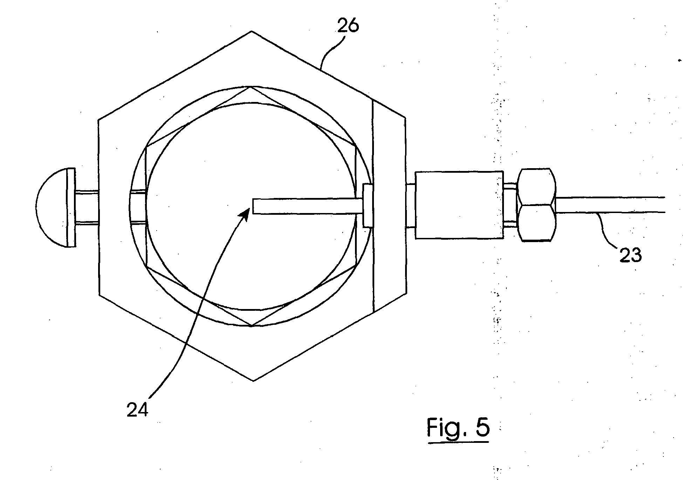

Inlet tube 23 is for introduction of the target solution/suspension, and is so shaped and located that the direction of flow of the solution/suspension at its outlet 24 (see Fig 5) will be perpendicular to that of the anti-solvent exiting nozzle 21. Again the tube is of circular cross section.

Fig 4 shows how tube 23 is mounted, by means of the supporting and locking pieces 25, on a collar 26 which is itself mounted around the lower portion of the nozzle 21. The arrangement is such as to allow adjustment of the distance "d" between the outlets of nozzle 21 and tube 23. It can be seen that the outlet of tube 23 is positioned on the central longitudinal axis of the nozzle 21.

Both the nozzle 21 and the tube 23 are preferably made from stainless steel.

The assembly of Figs 3 to 5 may be less likely to suffer blockages (at the nozzle and tube outlets) than a multi-component SEDS™ nozzle of the type described in WO-95/01221, particularly when the operating conditions are such as to allow a very rapid and efficient removal of the solvent vehicle, from the target solution/suspension, by the anti-solvent.

Examples

Apparatus as shown in Fig 2, incorporating a fluid inlet assembly as shown in Figs 3 to 5, was used to carry out particle formation methods in accordance with the invention. The nozzle 21 comprised a fluid inlet tube of internal diameter 1.6 mm and an outlet of diameter 0.2 mm. The internal bore at the end of the inlet tube 23 was 0.125 mm. The vertical separation "d" between the nozzle and tube outlets was varied between 0 and 8 mm, "0" representing the situation where the solution tube 23 contacted the lower end of the nozzle 21.

Supercritical carbon dioxide was used as the anti-solvent. It was pumped at a flow rate (of liquid $CO_2$, prior to passing through a heater) of 200 g/min. Its temperature on entry into the nozzle 21 was 356 K (83 °C).

The pressure in the particle formation vessel 1 (capacity 2 litres) was maintained at 80 bar and 309-313 K (36-40 °C). The $CO_2$ back pressure was between 250 and 300 bar. These conditions created a sonic or supersonic $CO_2$ velocity at the nozzle outlet 22.

*Examples A*

Various target compounds were dissolved in appropriate solvents and introduced into the apparatus via tube 23. The distance "d" between the outlets of the anti-solvent nozzle and the solution inlet tube was kept constant at 4 mm. Particle formation was allowed to occur by the action of the $CO_2$ anti-solvent, and the products collected in the vessel 1. The products were assessed by scanning electron microscopy (SEM) and in most cases their particle sizes analysed using an Aerosizer™ and/or Sympatec™ system.

The results of these experiments are shown in Table 1 below.

Table 1

| Expt no. | Target solution | Target solution concentration (% w/v) | Target solution flow rate (ml/min) | Product size (Aerosizer™) ($\mu$m) | Product size (Sympatec™) ($\mu$m) |
|---|---|---|---|---|---|
| A1 | Compound I in methanol | 3 | 4 | 2.84 | - |
| A2 | Compound II in methanol | 1.75 | 4 | - | 5.75 |
| A3 | Compound III in DMF | 3 | 0.5 | 1.39 | 7.99 |
| A4 | Compound IV in DMF | 0.85 | 4 | - | - |
| A5 | Compound V in DMSO | 3 | 1 | - | 4.6 |
| A6 | Compound VI in THF | 5 | 1 | 0.97 | 2.5 |

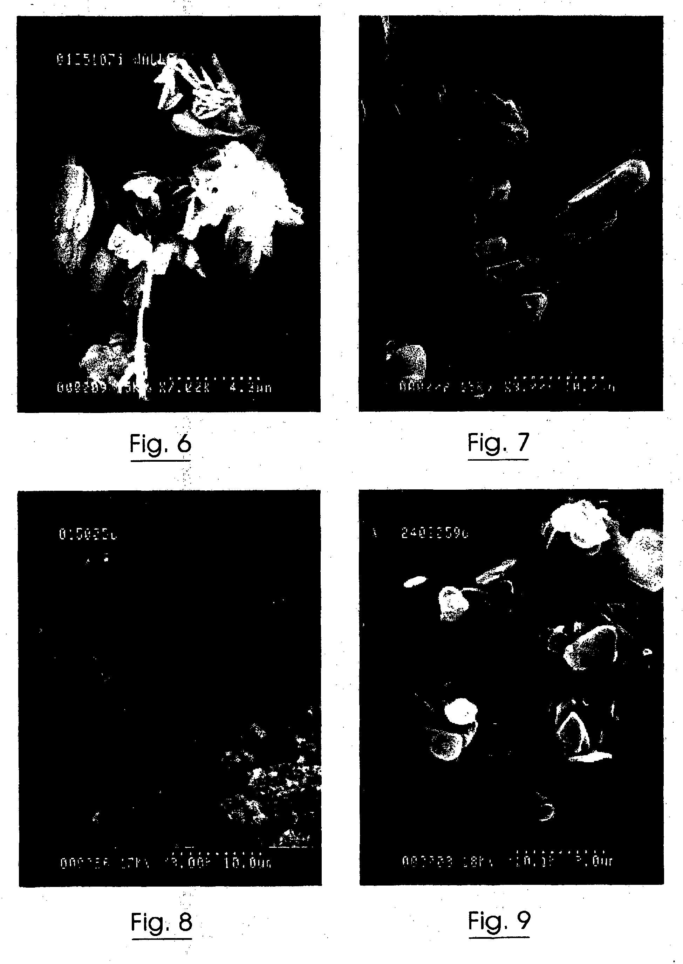

SEM photographs of the products of Experiments A1, A2, A5 and A6 are shown in Figs 6 to 9 respectively.

_Examples B_

In these experiments, the distance "d" between the outlets of the anti-solvent nozzle 21 and the solution inlet tube 23 was varied between 0 and 8 mm. In practice, the "0" separation represented the thickness of the inlet tube wall - in other words, as close to zero as was possible without cutting into the nozzle wall. The target solution was 3 % w/v compound I in methanol; its flow rate into the particle formation vessel 1 was 4 ml/min.
The results are shown in Table 2 below.

**Table 2**

| Expt no. | Distance "d" (mm) | Product size (Aerosizer™ ) (µm) |
|---|---|---|
| B1 | 0 | 3.21 |
| B2 | 4 | 2.84 |
| B3 | 8 | 3.63 |

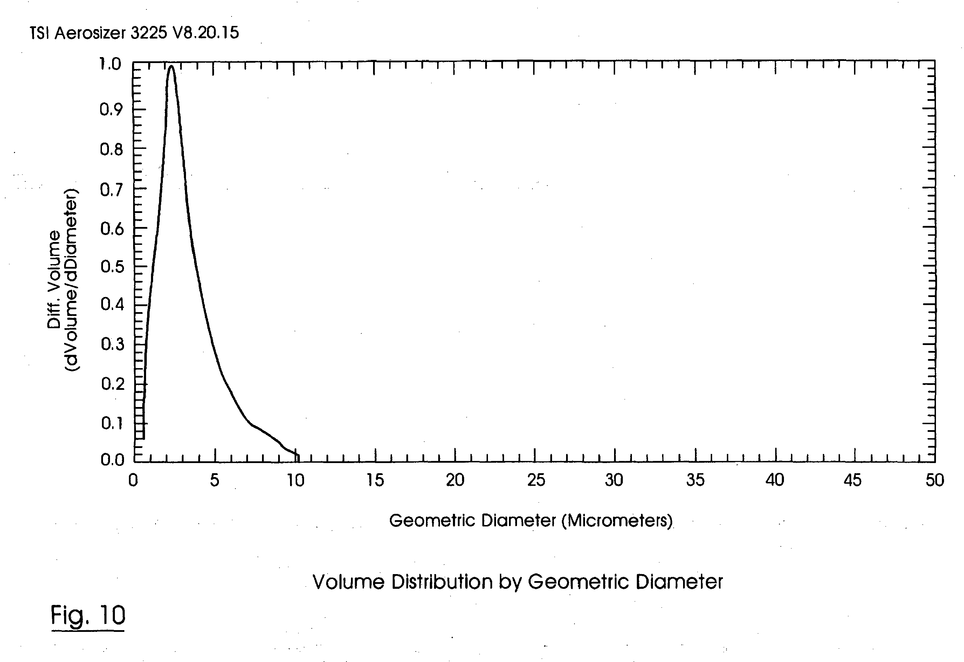

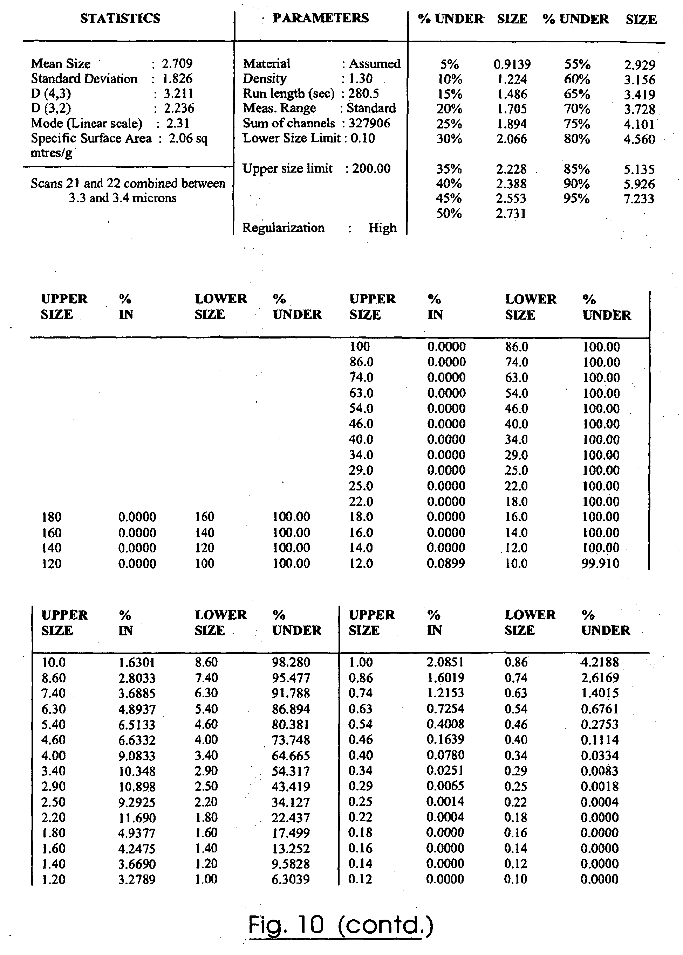

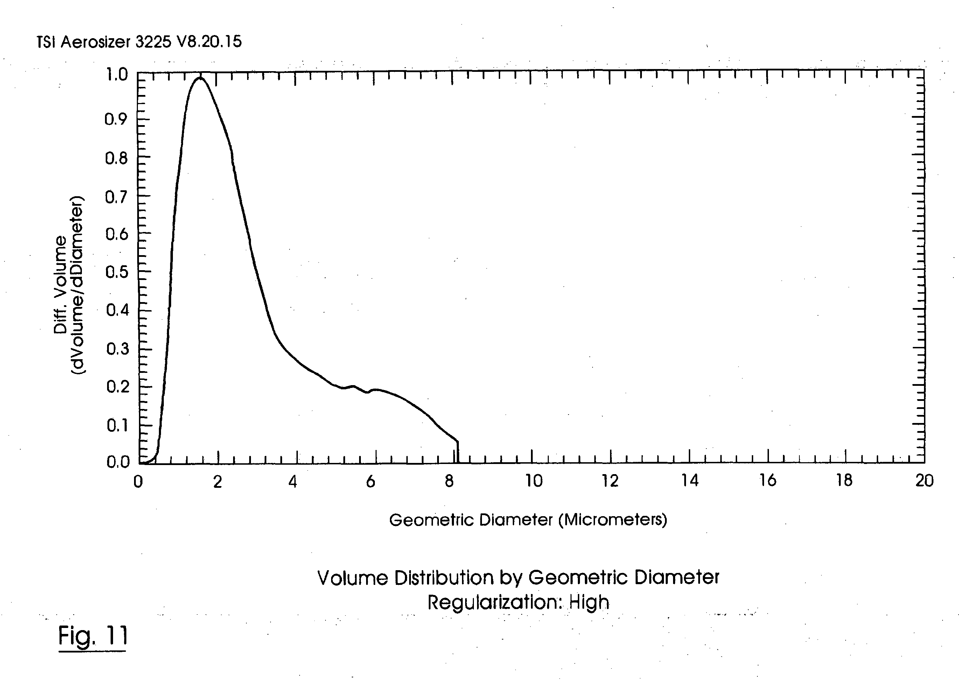

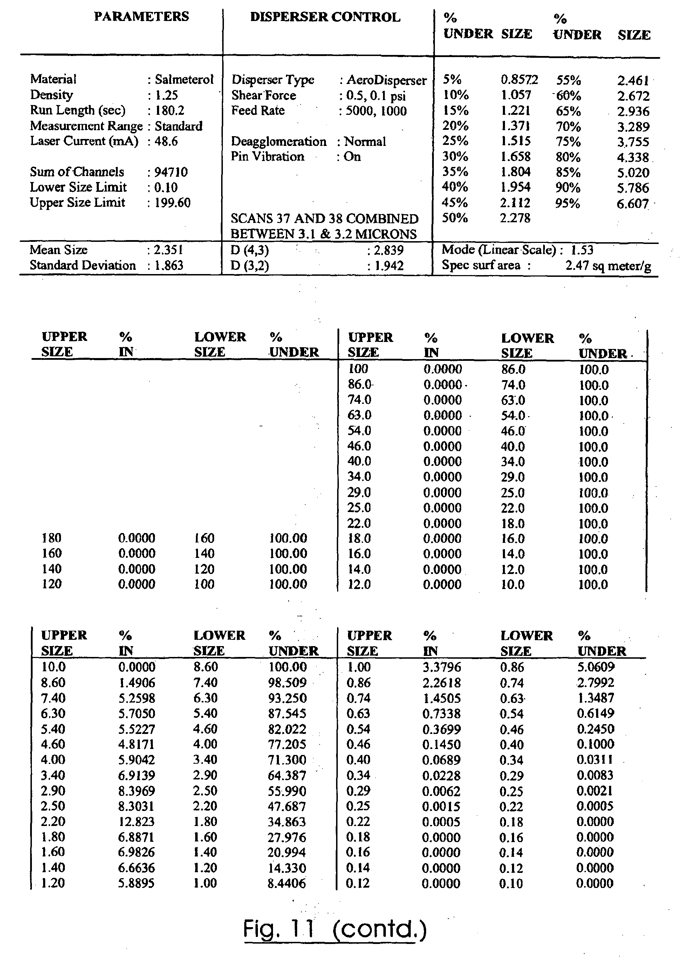

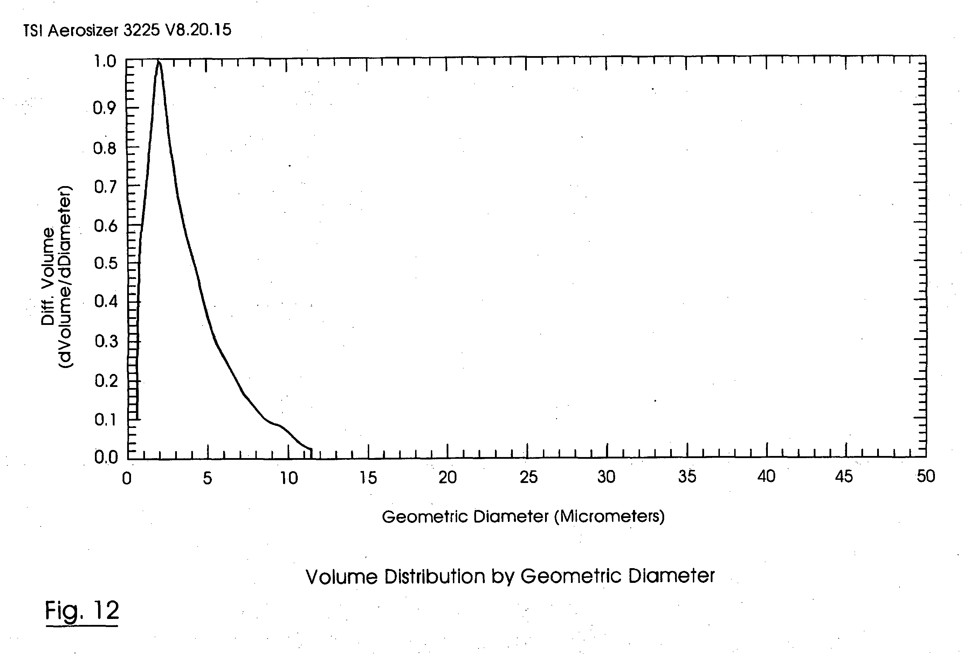

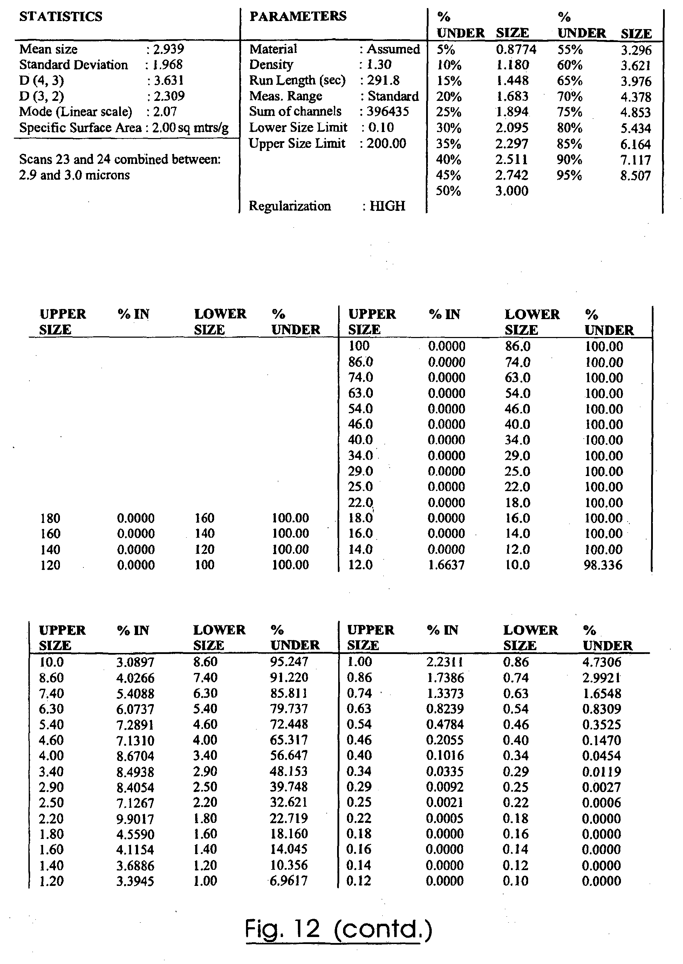

The particle size distributions (by Aerosizer™) for the products of Examples B1, B2 and B3 are shown in Figs 10 to 12 respectively.

_Examples C_

These experiments investigated the effect of the target solution flow rate on the product particle size. Again various target compounds were tested, the operating conditions being as for Examples A.
The results are given in Table 3 below.

**Table 3**

| Expt no | Target solution | Target solution concentration (% w/v) | Target solution flow rate (ml/min) | Product size (Aerosizer™) (µm) | Product size (Sympatec™) (µm) |
|---|---|---|---|---|---|
| C1 | Compound II in acetone | 0.75 | 2 | - | 7.8 |
| C2 | Compound II in acetone | 0.75 | 4 | - | 4.75 |
| C3 | Compound IV in DMF | 0.85 | 1 | - | - |
| C4 | Compound IV in DMF | 0.85 | 4 | - | - |
| C5 | Compound IV in DMF | 0.85 | 8 | - | - |
| C6 | Compound III in DMF | 3 | 0.5 | 1.39 | 7.99 |
| C7 | Compound III in DMF | 3 | 1.0 | 1.86 | 7.18 |
| C8 | Compound III in DMF | 3 | 4 | 18.18 | 10.5 |
| C9 | Compound V in DMF(ac)* | 1.6 | 1 | - | 9.1 |

(continued)

| Expt no. | Target solution | Target solution concentration (% w/v) | Target solution flow rate (ml/min) | Product size (Aerosizer™) ($\mu$m) | Product size (Sympatec™) ($\mu$m) |
|---|---|---|---|---|---|
| C10 | Compound V in DMF(ac)* | 1.6 | 4 | - | 42.3 |
| C11 | Compound VI in THF | 5 | 1 | 0.97 | 2.5 |
| C12 | Compound VI in THF | 5 | 4 | 1.18 | 3.0 |
| *DMF(ac) = DMF acidified with 4% v/v acetic acid | | | | | |

### Examples D

These experiments compared two types of fluid inlet assembly. In Example D1, a two-fluid coaxial nozzle of the type described in WO-95/01221 was used to co-introduce supercritical $CO_2$ and Compound VI in solution in THF (tetrahydrofuran). The internal diameter of the inner nozzle passage, through which the $CO_2$ was introduced, was 1.6 mm; that of the outer passage, through which the target solution was introduced, 2.5 mm. The nozzle outlet diameter was 0.2 mm.

In Example D2, an assembly of the type illustrated in Figs 3 to 5, with a nozzle outlet separation "d" of 4 mm, was used to introduce the same reagents. The $CO_2$ was introduced through the inner passage of the nozzle used in Example D1; the outer nozzle passage was not used.

All other operating conditions were the same for both experiments. Within the particle formation vessel the temperature was 309 K (36 °C) and the pressure was 80 bar. The target solution concentration was 5 % w/v and its flow rate 1 ml/min. The $CO_2$ flow rate was 200 g/min and its inlet temperature 356 K (83 °C).

The results are given in Table 4 below.

**Table 4**

| Expt no. | Product size (SEM) ($\mu$m) | Product size (Aerosizer™) ($\mu$m) |
|---|---|---|
| D1 | 1-6 $\mu$m | 2.54 |
| D2 | 750 nm - 4 $\mu$m | 1.5 |

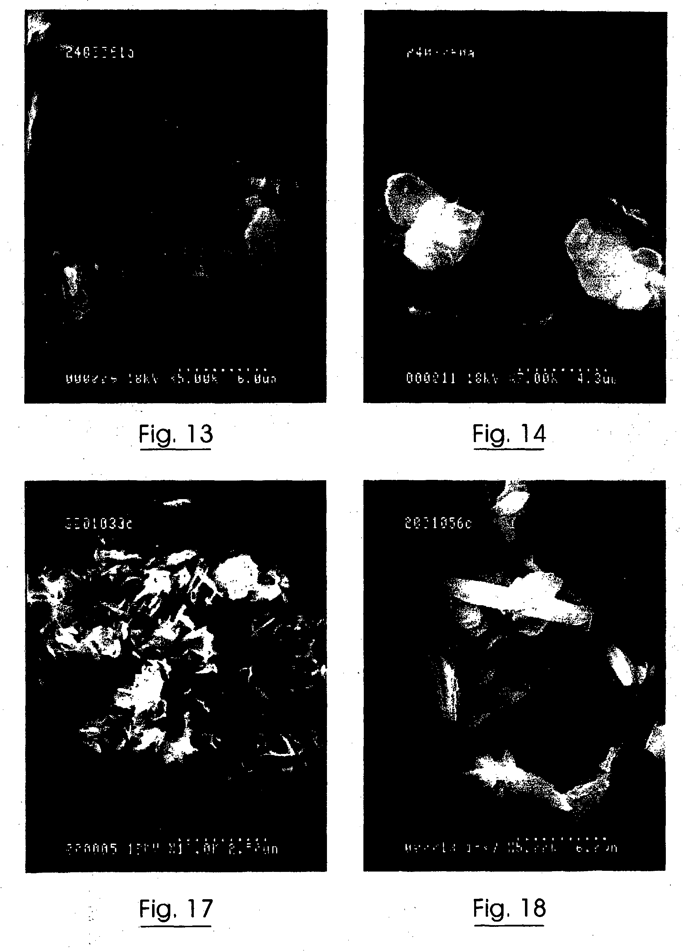

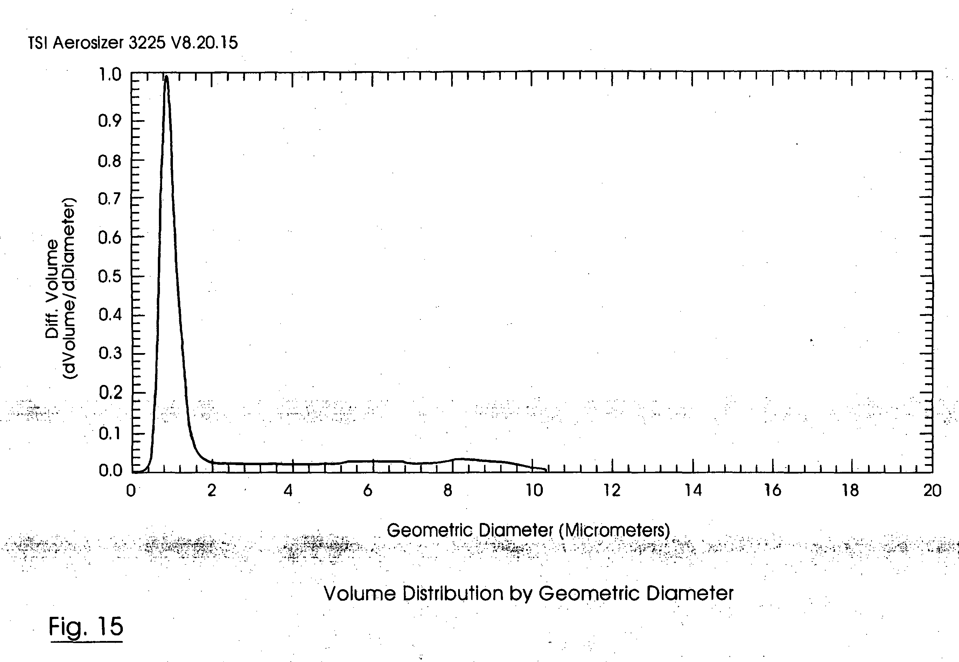

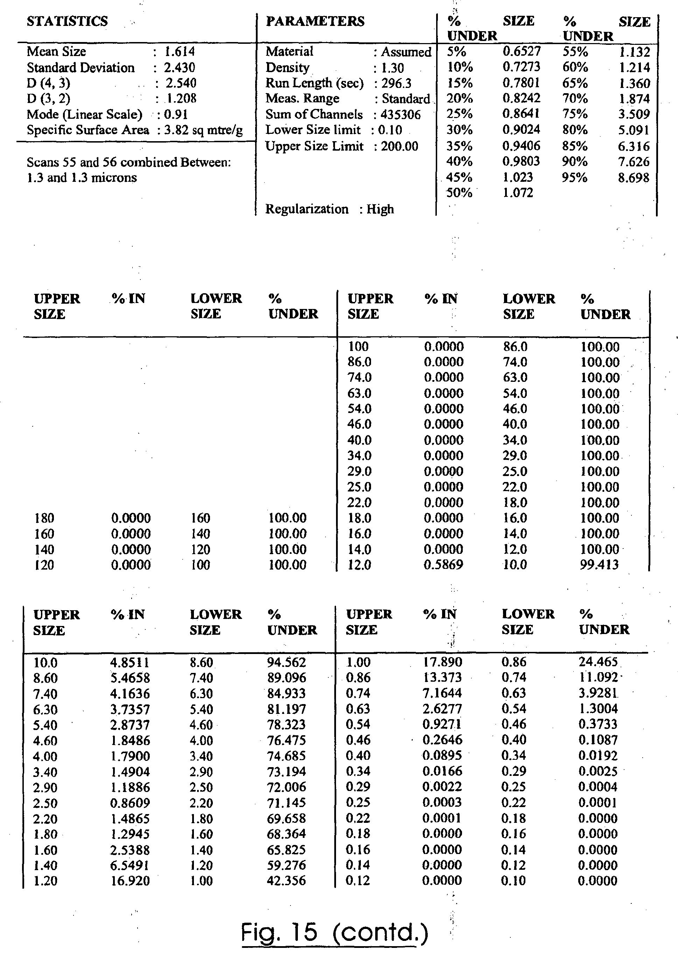

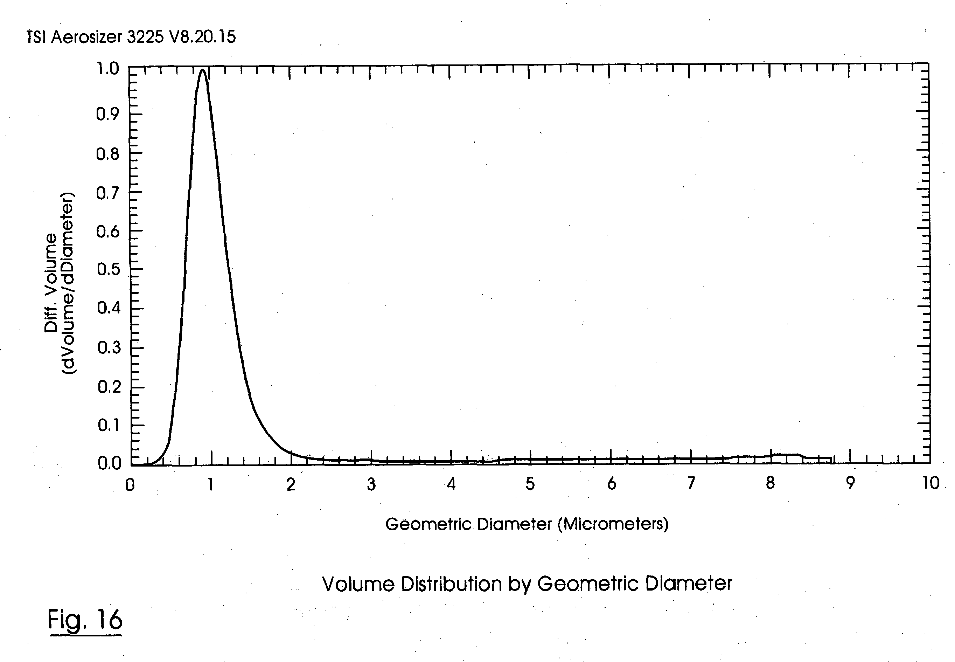

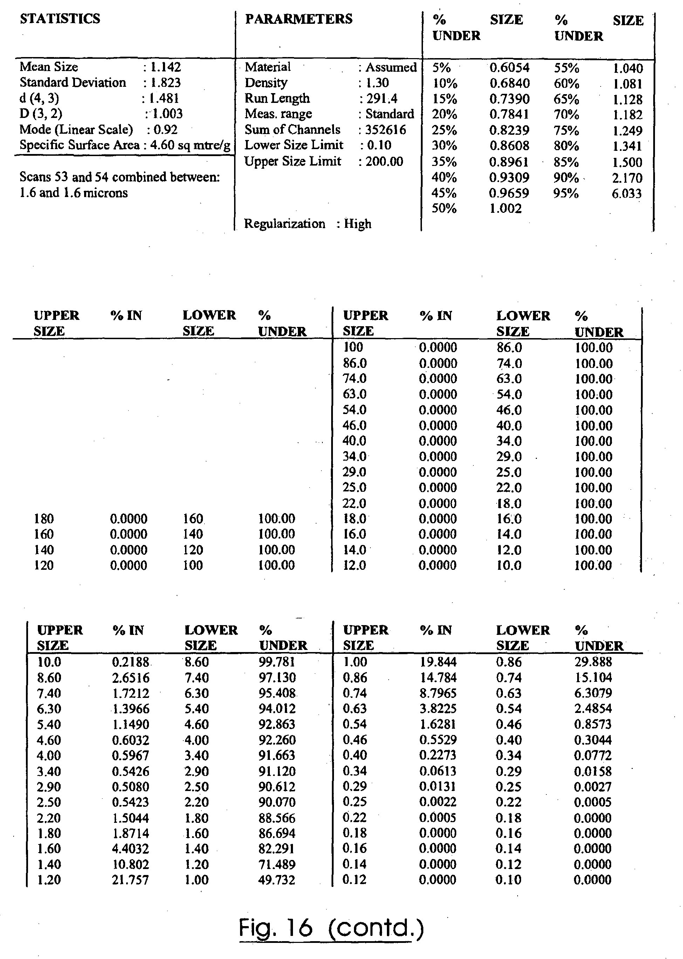

SEMs for the products of Examples D1 and D2 are shown in Figs 13 and 14 respectively. Their Aerosizer™ particle size distributions are shown in Figs 15 and 16 respectively, D2 showing a significantly smaller particle size and a better distribution than D1.

It was also found that the fluid inlet assembly of Figs 3 to 5 (Example D2) gave a less agglomerated product.

### Examples E

Two further target compounds, dihydroergotamine mesylate (Compound VII) and ipratropium bromide (Compound VIII) were prepared using a vessel temperature of 309 K (36 °C) and pressure of 80 bar, a $CO_2$ flow rate of 200 g/min and a nozzle separation "d" of 4 mm. The $CO_2$ temperature upstream of the vessel was 356 K (83 °C). Particle sizes were assessed using the Aerosizer™. The results are shown in Table 5 below.

**Table 5**

| Expt no. | Target solution | Target solution concentration (% w/v) | Target solution flow rate (ml/min) | $CO_2$ flow rate (ml/min) | Product size (Aerosizer™) ($\mu$m) |
|---|---|---|---|---|---|
| E1 | Compound VII in methanol | 4.0 | 1.0 | 200 | 6.78 |

(continued)

| Expt no. | Target solution | Target solution concentration (% w/v) | Target solution flow rate (ml/min) | CO$_2$ flow rate (ml/min) | Product size (Aerosizer™) (μm) |
|---|---|---|---|---|---|
| E2 | Compound VII in methanol:water (9:1 v/v) | 2.0 | 1.0 | 210 | 0.87 |
| E3 | Compound VIII in methanol:water (95:5 v/v) | 1.0 | 2.0 | 210 | 3.79 |
| E4 | Compound VIII in methanol:water (95:5 v/v) | 1.0 | 4.0 | 210 | 5.62 |

SEM photographs of the products of Experiments E2 and E3 are shown in Figs 17 and 18 respectively.

*Examples F*

Two drugs suitable for delivery by inhalation therapy were produced using the method of the invention. In all cases the products were fine, free-flowing powders having excellent dispersibility in fluids such as in particular the propellant fluids used to aerosolise such active substances in so-called "metered dose inhalers". The drugs exhibited improved flocculation performance in such propellants (in particular in HFA 134a and HFA 227ea), as compared to the performance of micronised versions of the same drugs having comparable particle sizes.

For these experiments, the CO$_2$ anti-solvent was pumped at different flow rates, as shown in Table 6 below. Its temperature on entry into the nozzle 21 of the Fig 2 apparatus was 363 K (90 °C). The pressure in the particle formation vessel 1 (capacity 2000 ml) was maintained at 80 bar and 309 K (36 °C). The vertical separation "d" between the nozzle and solution tube outlets was 4 mm.

The reagents, solvents and other relevant operating conditions are summarised in Table 6, together with the particle sizes and size distributions of the products.

**Table 6**

| Expt no. | Target substance | Vehicle | Target solution concn (% w/v) | Target solution flow rate (ml/min) | CO$_2$ flow rate (ml/min) | Product MMAD D(4,3) (μm) | Particle size spread |
|---|---|---|---|---|---|---|---|
| F1 | Salmeterol xinafoate | Methanol | 3 | 4 | 158 | 1.7 (A) | 1.8 (A) |
| F2 | Risperidone-(9-hydroxy)-palmitate | THF | 5 | 4 | 200 | 3.0 (S) | 1.52 (S) |
| F3 | Risperidone-(9-hydroxy)-palmitate | THF | 5 | 1 | 200 | 2.5 (S) | 1.52 (S) |

The particle sizes quoted in Table 6 are, where indicated (A), mass median aerodynamic diameters obtained using an Aerosizer™ time-of-flight instrument and, where indicated (S), geometric projection equivalent mass median diameters obtained using the Helos™ system available from Sympatec GmbH, Germany.

The particle size spread is defined as $(D_{90} - D_{10}) / D_{50}$ and indicates how narrow the size distribution may be for products made according to the present invention.

The flocculation behaviour of the products of Examples F, in the propellants HFA 134a and HFA 227ea, are documented in our co-pending UK patent application no. 0208742.7.

**Claims**

1. A method for preparing a target substance in particulate form, the method comprising introducing into a particle formation vessel, through separate first and second fluid inlet means respectively, (a) a solution or suspension of the target substance in a fluid vehicle i.e. the "target solution/suspension", and (b) a compressed fluid anti-solvent for the substance, and allowing the anti-solvent fluid to extract the vehicle from the target solution/suspension so as to form particles of the target substance, wherein the anti-solvent fluid has a sonic, near-sonic or supersonic velocity as it enters the particle formation vessel, and wherein the anti-solvent and the target solution/suspension enter the particle formation vessel at different locations and meet downstream, in the direction of anti-solvent flow, of the second fluid inlet means and the pressure drop as the anti-solvent fluid enters the particle formation vessel is from 170 to 250 bar.

2. A method according to claim 1, wherein the anti-solvent fluid is heated, upstream of the particle formation vessel, to a temperature sufficient to compensate for its Joule-Thomson cooling as it enters the vessel.

3. A method according to claim 1 or claim 2, wherein on entering the particle formation vessel, the anti-solvent fluid has a Mach number M (the ratio of its actual speed to the speed of sound) of from 0.8 to 1.5.

4. A method according to any one of the preceding claims, wherein the anti-solvent fluid and the target solution/suspension contact each other immediately downstream of the point of anti-solvent entry into the particle formation vessel.

5. Apparatus for use in preparing a target substance in particulate form, comprising:

    (i) a particle formation vessel;
    (ii) first fluid inlet means for introducing into the vessel a solution or suspension of the target substance in a fluid vehicle (the "target solution/suspension"); and
    (iii) second fluid inlet means, separate from the first, for introducing a compressed anti-solvent fluid into the particle formation vessel;

wherein the first and second fluid inlet means are so arranged that, in use, a target solution/suspension introduced through the first and an anti-solvent introduced through the second enter the particle formation vessel at different locations and meet immediately downstream, in the direction of anti-solvent flow, of the second fluid inlet means, and wherein the outlet of the first fluid inlet means is positioned such that, in use, it is directly within the flow of anti-solvent fluid exiting the second fluid inlet means.

6. Apparatus according to claim 5, wherein the outlet of the first fluid inlet means is downstream, in the direction of anti-solvent flow in use, of that of the second fluid inlet means, and wherein the separation between the two outlets is from 10 to 40 times the diameter of the outlet of the second fluid inlet means.

7. Apparatus according to claim 5 or claim 6, wherein the centre of the outlet of the first fluid inlet means is positioned in line with the central longitudinal axis of the second fluid inlet means.

8. Apparatus according to any one of claims 5 to 7, wherein the first and second fluid inlet means are arranged so that at the point where the target solution/suspension and the anti-solvent meet, the angle between their axes of flow is from 70 ° to 110 °.

**Patentansprüche**

1. Verfahren zum Herstellen einer Zielsubstanz in Teilchenform, umfassend das Einführen (a) einer Lösung oder Suspension der Zielsubstanz in einem Fluidvehikel, d.h. der "Ziellösung/Zielsuspension", und (b) eines komprimierten fluiden Nicht-Lösungsmittels für die Substanz in ein Teilchenbildungsgefäß durch jeweils getrennte erste und zweite Fluideinlassmittel, und das Ermöglichen der Extrahierung des Vehikels aus der Ziellösung/Zielsuspension durch das Nicht-Lösungsmittelfluid, um somit Teilchen der Zielsubstanz zu bilden, wobei das Nicht-Lösungsmittelfluid Schallgeschwindigkeit, annähernd Schallgeschwindigkeit oder Überschallgeschwindigkeit aufweist, wenn es in das Teilchenbildungsgefäß eintritt, und wobei das Nicht-Lösungsmittel und die Ziellösung/Zielsuspension an verschiedenen Orten in das Teilchenbildungsgefäß eintreten und in Richtung des Nicht-Lösungsmittelstroms stromabwärts des zweiten Fluideinlassmittels aufeinander treffen und der Druckabfall bei Eintritt des Nicht-Lösungsmittelfluids in das Teilchenbildungsgefäß bei 170 bis 250 bar liegt.

2. Verfahren nach Anspruch 1, wobei das Nicht-Lösungsmittelfluid stromaufwärts des Teilchenbildungsgefäßes auf eine Temperatur erhitzt wird, die ausreichend ist, um dessen Joule-Thomson-Abkühlung beim Eintritt in das Gefäß auszugleichen.

3. Verfahren nach Anspruch 1 oder 2, wobei das Nicht-Lösungsmittel bei Eintritt in das Teilchenbildungsgefäß eine Machzahl M (das Verhältnis seiner tatsächlichen Geschwindigkeit zur Schallgeschwindigkeit) von 0,8 bis 1,5 aufweist.

4. Verfahren nach einem der vorhergehenden Ansprüche, wobei das Nicht-Lösungsmittelfluid und die Ziellösung/Zielsuspension unmittelbar stromabwärts des Eintrittspunkts des Nicht-Lösungsmittels in das Teilchenbildungsgefäß miteinander in Kontakt kommen.

5. Vorrichtung zur Verwendung bei der Herstellung einer Zielsubstanz in Teilchenform, umfassend:

   (i) ein Teilchenbildungsgefäß;
   (ii) ein erstes Fluideinlassmittel zum Einführen einer Lötung oder Suspension der Zielsubstanz in einem Fluidvehikel (die "Ziellösung/Zielsuspension") in das Gefäß; und
   (iii) ein zweites Fluideinlassmittel, getrennt von dem ersten, zum Einführen eines komprimierten Nicht-Lösungsmittelfluids in das Teilchenbildungsgefäß;

   wobei die ersten und zweiten Fluideinlassmittel derart angeordnet sind, dass bei der Verwendung eine durch das erste Einlassmittel eingeführte Ziellösung/Zielsuspension und ein durch das zweite Einlassmittel eingeführtes Nicht-Lösungsmittel an verschiedenen Orten in das Teilchenbildungsgefäß eintreten und in Richtung des Nicht-Lösungsmittelstroms unmittelbar stromabwärts des zweiten Fluideinlassmittels aufeinandertreffen, und wobei der Auslass des ersten Fluideinlassmittels derart positioniert ist, dass er bei der Verwendung direkt innerhalb des Stroms des aus dem zweiten Fluideinlassmittel austretenden Nicht-Lösungsmittelfluids liegt.

6. Vorrichtung nach Anspruch 5, wobei sich der Auslass des ersten Fluideinlassmittels in Richtung des Nicht-Lösungsmittelstroms in Verwendung stromabwärts des Auslasses des zweiten Fluideinlassmittels befindet, und wobei die Trennung der zwei Auslässe das Zehn- bis Vierzigfache des Durchmessers des Auslasses des zweiten Fluideinlassmittels beträgt.

7. Vorrichtung nach Anspruch 5 oder Anspruch 6, wobei der Mittelpunkt des Auslasses des ersten Fluideinlassmittels in Ausrichtung mit der Längsmittelachse des zweiten Fluideinlassmittels positioniert ist.

8. Vorrichtung nach einem der Ansprüche 5 bis 7, wobei die ersten und zweiten Fluideinlassmittel derart angeordnet sind, dass an der Stelle, an der die Ziellösung/Zielsuspension und das Nicht-Lösungsmittel aufeinandertreffen, der Winkel zwischen ihren Strömungsachsen 70° bis 110° beträgt.

## Revendications

1. Méthode de préparation d'une substance cible sous forme particulaire, la méthode comprenant l'introduction dans un récipient de formation de particules, respectivement par un premier moyen et un second moyen d'entrée de fluide, (a) d'une solution ou d'une suspension de la substance cible dans un véhicule de fluide, à savoir la « solution/suspension cible » et (b) d'un fluide comprimé anti-solvant pour la substance et le fait de permettre au fluide anti-solvant d'extraire le véhicule de la solution/suspension cible de façon à former des particules de la substance cible, dans laquelle le fluide anti-solvant a une vitesse sonique, pratiquement sonique ou supersonique lorsqu'il entre dans le récipient de formation de particules et dans laquelle l'anti-solvant et la solution/suspension cible entrent dans le récipient de formation de particules en des positions différentes et se rencontrent en aval, dans le sens d'écoulement de l'anti-solvant, du second moyen d'entrée de fluide et la perte de charge lorsque le fluide anti-solvant entre dans le récipient de formation de particules est de 170 à 250 bars.

2. Méthode selon la revendication 1, dans laquelle le fluide anti-solvant est chauffé, en amont du récipient de formation de particules, jusqu'à une température suffisante pour compenser son refroidissement Joule-Thomson lorsqu'il entre dans le récipient.

3. Méthode selon la revendication 1 ou la revendication 2, dans laquelle, lors de son entrée dans le récipient de formation de particules, le fluide anti-solvant a un nombre de Mach M (le rapport de sa vitesse réelle à la vitesse du son) de 0,8 à 1,5.

**4.** Méthode selon l'une quelconque des revendications précédentes, dans laquelle le fluide anti-solvant et la solution/suspension cible entrent en contact l'un avec l'autre immédiatement en aval du point d'entrée de l'anti-solvant dans le récipient de formation de particules.

**5.** Appareil à utiliser pour préparer une substance cible sous forme particulaire, comprenant :

(i) un récipient de formation de particules ;
(ii) un premier moyen d'entrée de fluide pour introduire dans le récipient une solution ou une suspension de la substance cible dans un véhicule de fluide (la « solution/suspension cible ») ; et
(iii) un second moyen d'entrée de fluide, séparé du premier, pour introduire un fluide comprimé anti-solvant dans le récipient de formation de particules ;

dans lequel le premier moyen et le second moyen d'entrée de fluide sont disposés de telle sorte qu'à l'emploi, une solution/suspension cible introduite par le premier et un anti-solvant introduit par le second entrent dans le récipient de formation de particules en différentes positions et se rencontrent immédiatement en aval, dans le sens d'écoulement de l'anti-solvant, du second moyen d'entrée de fluide, et dans lequel la sortie du premier moyen d'entrée de fluide est placée de sorte qu'à l'emploi, elle soit directement dans le flux du fluide anti-solvant sortant du second moyen d'entrée de fluide.

**6.** Appareil selon la revendication 5, dans lequel la sortie du premier moyen d'entrée de fluide est en aval, dans le sens d'écoulement de l'anti-solvant, à l'emploi, de celle du second moyen d'entrée de fluide et dans lequel la séparation entre les deux sorties est de 10 à 40 fois le diamètre de la sortie du second moyen d'entrée de fluide.

**7.** Appareil selon la revendication 5 ou la revendication 6, dans lequel le centre de la sortie du premier moyen d'entrée de fluide est placé en ligne avec l'axe longitudinal central du second moyen d'entrée de fluide.

**8.** Appareil selon l'une quelconque des revendications 5 à 7, dans lequel le premier moyen et le second moyen d'entrée de fluide sont disposés de telle sorte qu'au point où la solution/suspension cible et l'anti-solvant se rencontrent, l'angle entre leurs axes d'écoulement est de 70° à 110°.

Fig. 1  Enthalpy variation of Carbon dioxide with temperature and pressure

17

Fig. 2

anti-solvent    Solution

Fig. 3

Fig. 4

Fig. 5

Fig. 6

Fig. 7

Fig. 8

Fig. 9

TSI Aerosizer 3225 V8.20.15

Volume Distribution by Geometric Diameter

Fig. 10

EP 1 409 123 B1

| STATISTICS | | PARAMETERS | | % UNDER | SIZE | % UNDER | SIZE |
|---|---|---|---|---|---|---|---|
| Mean Size | : 2.709 | Material | : Assumed | 5% | 0.9139 | 55% | 2.929 |
| Standard Deviation | : 1.826 | Density | : 1.30 | 10% | 1.224 | 60% | 3.156 |
| D (4,3) | : 3.211 | Run length (sec) | : 280.5 | 15% | 1.486 | 65% | 3.419 |
| D (3,2) | : 2.236 | Meas. Range | : Standard | 20% | 1.705 | 70% | 3.728 |
| Mode (Linear scale) | : 2.31 | Sum of channels | : 327906 | 25% | 1.894 | 75% | 4.101 |
| Specific Surface Area | : 2.06 sq | Lower Size Limit | : 0.10 | 30% | 2.066 | 80% | 4.560 |
| mtres/g | | | | | | | |
| | | Upper size limit | : 200.00 | 35% | 2.228 | 85% | 5.135 |
| Scans 21 and 22 combined between | | | | 40% | 2.388 | 90% | 5.926 |
| 3.3 and 3.4 microns | | | | 45% | 2.553 | 95% | 7.233 |
| | | | | 50% | 2.731 | | |
| | | Regularization | : High | | | | |

| UPPER SIZE | % IN | LOWER SIZE | % UNDER | UPPER SIZE | % IN | LOWER SIZE | % UNDER |
|---|---|---|---|---|---|---|---|
| | | | | 100 | 0.0000 | 86.0 | 100.00 |
| | | | | 86.0 | 0.0000 | 74.0 | 100.00 |
| | | | | 74.0 | 0.0000 | 63.0 | 100.00 |
| | | | | 63.0 | 0.0000 | 54.0 | 100.00 |
| | | | | 54.0 | 0.0000 | 46.0 | 100.00 |
| | | | | 46.0 | 0.0000 | 40.0 | 100.00 |
| | | | | 40.0 | 0.0000 | 34.0 | 100.00 |
| | | | | 34.0 | 0.0000 | 29.0 | 100.00 |
| | | | | 29.0 | 0.0000 | 25.0 | 100.00 |
| | | | | 25.0 | 0.0000 | 22.0 | 100.00 |
| | | | | 22.0 | 0.0000 | 18.0 | 100.00 |
| 180 | 0.0000 | 160 | 100.00 | 18.0 | 0.0000 | 16.0 | 100.00 |
| 160 | 0.0000 | 140 | 100.00 | 16.0 | 0.0000 | 14.0 | 100.00 |
| 140 | 0.0000 | 120 | 100.00 | 14.0 | 0.0000 | 12.0 | 100.00 |
| 120 | 0.0000 | 100 | 100.00 | 12.0 | 0.0899 | 10.0 | 99.910 |

| UPPER SIZE | % IN | LOWER SIZE | % UNDER | UPPER SIZE | % IN | LOWER SIZE | % UNDER |
|---|---|---|---|---|---|---|---|
| 10.0 | 1.6301 | 8.60 | 98.280 | 1.00 | 2.0851 | 0.86 | 4.2188 |
| 8.60 | 2.8033 | 7.40 | 95.477 | 0.86 | 1.6019 | 0.74 | 2.6169 |
| 7.40 | 3.6885 | 6.30 | 91.788 | 0.74 | 1.2153 | 0.63 | 1.4015 |
| 6.30 | 4.8937 | 5.40 | 86.894 | 0.63 | 0.7254 | 0.54 | 0.6761 |
| 5.40 | 6.5133 | 4.60 | 80.381 | 0.54 | 0.4008 | 0.46 | 0.2753 |
| 4.60 | 6.6332 | 4.00 | 73.748 | 0.46 | 0.1639 | 0.40 | 0.1114 |
| 4.00 | 9.0833 | 3.40 | 64.665 | 0.40 | 0.0780 | 0.34 | 0.0334 |
| 3.40 | 10.348 | 2.90 | 54.317 | 0.34 | 0.0251 | 0.29 | 0.0083 |
| 2.90 | 10.898 | 2.50 | 43.419 | 0.29 | 0.0065 | 0.25 | 0.0018 |
| 2.50 | 9.2925 | 2.20 | 34.127 | 0.25 | 0.0014 | 0.22 | 0.0004 |
| 2.20 | 11.690 | 1.80 | 22.437 | 0.22 | 0.0004 | 0.18 | 0.0000 |
| 1.80 | 4.9377 | 1.60 | 17.499 | 0.18 | 0.0000 | 0.16 | 0.0000 |
| 1.60 | 4.2475 | 1.40 | 13.252 | 0.16 | 0.0000 | 0.14 | 0.0000 |
| 1.40 | 3.6690 | 1.20 | 9.5828 | 0.14 | 0.0000 | 0.12 | 0.0000 |
| 1.20 | 3.2789 | 1.00 | 6.3039 | 0.12 | 0.0000 | 0.10 | 0.0000 |

## Fig. 10 (contd.)

Fig. 11

| PARAMETERS | | DISPERSER CONTROL | | % UNDER | SIZE | % UNDER | SIZE |
|---|---|---|---|---|---|---|---|
| Material | : Salmeterol | Disperser Type | : AeroDisperser | 5% | 0.8572 | 55% | 2.461 |
| Density | : 1.25 | Shear Force | : 0.5, 0.1 psi | 10% | 1.057 | 60% | 2.672 |
| Run Length (sec) | : 180.2 | Feed Rate | : 5000, 1000 | 15% | 1.221 | 65% | 2.936 |
| Measurement Range | : Standard | | | 20% | 1.371 | 70% | 3.289 |
| Laser Current (mA) | : 48.6 | Deagglomeration | : Normal | 25% | 1.515 | 75% | 3.755 |
| | | Pin Vibration | : On | 30% | 1.658 | 80% | 4.338 |
| Sum of Channels | : 94710 | | | 35% | 1.804 | 85% | 5.020 |
| Lower Size Limit | : 0.10 | | | 40% | 1.954 | 90% | 5.786 |
| Upper Size Limit | : 199.60 | | | 45% | 2.112 | 95% | 6.607 |
| | | SCANS 37 AND 38 COMBINED BETWEEN 3.1 & 3.2 MICRONS | | 50% | 2.278 | | |
| Mean Size | : 2.351 | D (4,3) | : 2.839 | Mode (Linear Scale) : 1.53 | | | |
| Standard Deviation | : 1.863 | D (3,2) | : 1.942 | Spec surf area : | | 2.47 sq meter/g | |

| UPPER SIZE | % IN | LOWER SIZE | % UNDER | UPPER SIZE | % IN | LOWER SIZE | % UNDER |
|---|---|---|---|---|---|---|---|
| | | | | 100 | 0.0000 | 86.0 | 100.0 |
| | | | | 86.0 | 0.0000 | 74.0 | 100.0 |
| | | | | 74.0 | 0.0000 | 63.0 | 100.0 |
| | | | | 63.0 | 0.0000 | 54.0 | 100.0 |
| | | | | 54.0 | 0.0000 | 46.0 | 100.0 |
| | | | | 46.0 | 0.0000 | 40.0 | 100.0 |
| | | | | 40.0 | 0.0000 | 34.0 | 100.0 |
| | | | | 34.0 | 0.0000 | 29.0 | 100.0 |
| | | | | 29.0 | 0.0000 | 25.0 | 100.0 |
| | | | | 25.0 | 0.0000 | 22.0 | 100.0 |
| | | | | 22.0 | 0.0000 | 18.0 | 100.0 |
| 180 | 0.0000 | 160 | 100.00 | 18.0 | 0.0000 | 16.0 | 100.0 |
| 160 | 0.0000 | 140 | 100.00 | 16.0 | 0.0000 | 14.0 | 100.0 |
| 140 | 0.0000 | 120 | 100.00 | 14.0 | 0.0000 | 12.0 | 100.0 |
| 120 | 0.0000 | 100 | 100.00 | 12.0 | 0.0000 | 10.0 | 100.0 |

| UPPER SIZE | % IN | LOWER SIZE | % UNDER | UPPER SIZE | % IN | LOWER SIZE | % UNDER |
|---|---|---|---|---|---|---|---|
| 10.0 | 0.0000 | 8.60 | 100.00 | 1.00 | 3.3796 | 0.86 | 5.0609 |
| 8.60 | 1.4906 | 7.40 | 98.509 | 0.86 | 2.2618 | 0.74 | 2.7992 |
| 7.40 | 5.2598 | 6.30 | 93.250 | 0.74 | 1.4505 | 0.63 | 1.3487 |
| 6.30 | 5.7050 | 5.40 | 87.545 | 0.63 | 0.7338 | 0.54 | 0.6149 |
| 5.40 | 5.5227 | 4.60 | 82.022 | 0.54 | 0.3699 | 0.46 | 0.2450 |
| 4.60 | 4.8171 | 4.00 | 77.205 | 0.46 | 0.1450 | 0.40 | 0.1000 |
| 4.00 | 5.9042 | 3.40 | 71.300 | 0.40 | 0.0689 | 0.34 | 0.0311 |
| 3.40 | 6.9139 | 2.90 | 64.387 | 0.34 | 0.0228 | 0.29 | 0.0083 |
| 2.90 | 8.3969 | 2.50 | 55.990 | 0.29 | 0.0062 | 0.25 | 0.0021 |
| 2.50 | 8.3031 | 2.20 | 47.687 | 0.25 | 0.0015 | 0.22 | 0.0005 |
| 2.20 | 12.823 | 1.80 | 34.863 | 0.22 | 0.0005 | 0.18 | 0.0000 |
| 1.80 | 6.8871 | 1.60 | 27.976 | 0.18 | 0.0000 | 0.16 | 0.0000 |
| 1.60 | 6.9826 | 1.40 | 20.994 | 0.16 | 0.0000 | 0.14 | 0.0000 |
| 1.40 | 6.6636 | 1.20 | 14.330 | 0.14 | 0.0000 | 0.12 | 0.0000 |
| 1.20 | 5.8895 | 1.00 | 8.4406 | 0.12 | 0.0000 | 0.10 | 0.0000 |

## Fig. 11 (contd.)

TSI Aerosizer 3225 V8.20.15

Volume Distribution by Geometric Diameter

Fig. 12

| STATISTICS | | PARAMETERS | | % UNDER | SIZE | % UNDER | SIZE |
|---|---|---|---|---|---|---|---|
| Mean size | : 2.939 | Material | : Assumed | 5% | 0.8774 | 55% | 3.296 |
| Standard Deviation | : 1.968 | Density | : 1.30 | 10% | 1.180 | 60% | 3.621 |
| D (4, 3) | : 3.631 | Run Length (sec) | : 291.8 | 15% | 1.448 | 65% | 3.976 |
| D (3, 2) | : 2.309 | Meas. Range | : Standard | 20% | 1.683 | 70% | 4.378 |
| Mode (Linear scale) | : 2.07 | Sum of channels | : 396435 | 25% | 1.894 | 75% | 4.853 |
| Specific Surface Area | : 2.00 sq mtrs/g | Lower Size Limit | : 0.10 | 30% | 2.095 | 80% | 5.434 |
| | | Upper Size Limit | : 200.00 | 35% | 2.297 | 85% | 6.164 |
| Scans 23 and 24 combined between: | | | | 40% | 2.511 | 90% | 7.117 |
| 2.9 and 3.0 microns | | | | 45% | 2.742 | 95% | 8.507 |
| | | | | 50% | 3.000 | | |
| | | Regularization | : HIGH | | | | |

| UPPER SIZE | % IN | LOWER SIZE | % UNDER | UPPER SIZE | % IN | LOWER SIZE | % UNDER |
|---|---|---|---|---|---|---|---|
| | | | | 100 | 0.0000 | 86.0 | 100.00 |
| | | | | 86.0 | 0.0000 | 74.0 | 100.00 |
| | | | | 74.0 | 0.0000 | 63.0 | 100.00 |
| | | | | 63.0 | 0.0000 | 54.0 | 100.00 |
| | | | | 54.0 | 0.0000 | 46.0 | 100.00 |
| | | | | 46.0 | 0.0000 | 40.0 | 100.00 |
| | | | | 40.0 | 0.0000 | 34.0 | 100.00 |
| | | | | 34.0 | 0.0000 | 29.0 | 100.00 |
| | | | | 29.0 | 0.0000 | 25.0 | 100.00 |
| | | | | 25.0 | 0.0000 | 22.0 | 100.00 |
| | | | | 22.0 | 0.0000 | 18.0 | 100.00 |
| 180 | 0.0000 | 160 | 100.00 | 18.0 | 0.0000 | 16.0 | 100.00 |
| 160 | 0.0000 | 140 | 100.00 | 16.0 | 0.0000 | 14.0 | 100.00 |
| 140 | 0.0000 | 120 | 100.00 | 14.0 | 0.0000 | 12.0 | 100.00 |
| 120 | 0.0000 | 100 | 100.00 | 12.0 | 1.6637 | 10.0 | 98.336 |

| UPPER SIZE | % IN | LOWER SIZE | % UNDER | UPPER SIZE | % IN | LOWER SIZE | % UNDER |
|---|---|---|---|---|---|---|---|
| 10.0 | 3.0897 | 8.60 | 95.247 | 1.00 | 2.2311 | 0.86 | 4.7306 |
| 8.60 | 4.0266 | 7.40 | 91.220 | 0.86 | 1.7386 | 0.74 | 2.9921 |
| 7.40 | 5.4088 | 6.30 | 85.811 | 0.74 | 1.3373 | 0.63 | 1.6548 |
| 6.30 | 6.0737 | 5.40 | 79.737 | 0.63 | 0.8239 | 0.54 | 0.8309 |
| 5.40 | 7.2891 | 4.60 | 72.448 | 0.54 | 0.4784 | 0.46 | 0.3525 |
| 4.60 | 7.1310 | 4.00 | 65.317 | 0.46 | 0.2055 | 0.40 | 0.1470 |
| 4.00 | 8.6704 | 3.40 | 56.647 | 0.40 | 0.1016 | 0.34 | 0.0454 |
| 3.40 | 8.4938 | 2.90 | 48.153 | 0.34 | 0.0335 | 0.29 | 0.0119 |
| 2.90 | 8.4054 | 2.50 | 39.748 | 0.29 | 0.0092 | 0.25 | 0.0027 |
| 2.50 | 7.1267 | 2.20 | 32.621 | 0.25 | 0.0021 | 0.22 | 0.0006 |
| 2.20 | 9.9017 | 1.80 | 22.719 | 0.22 | 0.0005 | 0.18 | 0.0000 |
| 1.80 | 4.5590 | 1.60 | 18.160 | 0.18 | 0.0000 | 0.16 | 0.0000 |
| 1.60 | 4.1154 | 1.40 | 14.045 | 0.16 | 0.0000 | 0.14 | 0.0000 |
| 1.40 | 3.6886 | 1.20 | 10.356 | 0.14 | 0.0000 | 0.12 | 0.0000 |
| 1.20 | 3.3945 | 1.00 | 6.9617 | 0.12 | 0.0000 | 0.10 | 0.0000 |

## Fig. 12 (contd.)

Fig. 13

Fig. 14

Fig. 17

Fig. 18

Fig. 15

Volume Distribution by Geometric Diameter

| STATISTICS | | PARAMETERS | | % UNDER | SIZE | % UNDER | SIZE |
|---|---|---|---|---|---|---|---|
| Mean Size | : 1.614 | Material | : Assumed | 5% | 0.6527 | 55% | 1.132 |
| Standard Deviation | : 2.430 | Density | : 1.30 | 10% | 0.7273 | 60% | 1.214 |
| D (4, 3) | : 2.540 | Run Length (sec) | : 296.3 | 15% | 0.7801 | 65% | 1.360 |
| D (3, 2) | : 1.208 | Meas. Range | : Standard | 20% | 0.8242 | 70% | 1.874 |
| Mode (Linear Scale) | : 0.91 | Sum of Channels | : 435306 | 25% | 0.8641 | 75% | 3.509 |
| Specific Surface Area | : 3.82 sq mtre/g | Lower Size limit | : 0.10 | 30% | 0.9024 | 80% | 5.091 |
| | | Upper Size Limit | : 200.00 | 35% | 0.9406 | 85% | 6.316 |
| Scans 55 and 56 combined Between: | | | | 40% | 0.9803 | 90% | 7.626 |
| 1.3 and 1.3 microns | | | | 45% | 1.023 | 95% | 8.698 |
| | | | | 50% | 1.072 | | |
| | | Regularization | : High | | | | |

| UPPER SIZE | % IN | LOWER SIZE | % UNDER | UPPER SIZE | % IN | LOWER SIZE | % UNDER |
|---|---|---|---|---|---|---|---|
| | | | | 100 | 0.0000 | 86.0 | 100.00 |
| | | | | 86.0 | 0.0000 | 74.0 | 100.00 |
| | | | | 74.0 | 0.0000 | 63.0 | 100.00 |
| | | | | 63.0 | 0.0000 | 54.0 | 100.00 |
| | | | | 54.0 | 0.0000 | 46.0 | 100.00 |
| | | | | 46.0 | 0.0000 | 40.0 | 100.00 |
| | | | | 40.0 | 0.0000 | 34.0 | 100.00 |
| | | | | 34.0 | 0.0000 | 29.0 | 100.00 |
| | | | | 29.0 | 0.0000 | 25.0 | 100.00 |
| | | | | 25.0 | 0.0000 | 22.0 | 100.00 |
| | | | | 22.0 | 0.0000 | 18.0 | 100.00 |
| 180 | 0.0000 | 160 | 100.00 | 18.0 | 0.0000 | 16.0 | 100.00 |
| 160 | 0.0000 | 140 | 100.00 | 16.0 | 0.0000 | 14.0 | 100.00 |
| 140 | 0.0000 | 120 | 100.00 | 14.0 | 0.0000 | 12.0 | 100.00 |
| 120 | 0.0000 | 100 | 100.00 | 12.0 | 0.5869 | 10.0 | 99.413 |

| UPPER SIZE | % IN | LOWER SIZE | % UNDER | UPPER SIZE | % IN | LOWER SIZE | % UNDER |
|---|---|---|---|---|---|---|---|
| 10.0 | 4.8511 | 8.60 | 94.562 | 1.00 | 17.890 | 0.86 | 24.465 |
| 8.60 | 5.4658 | 7.40 | 89.096 | 0.86 | 13.373 | 0.74 | 11.092 |
| 7.40 | 4.1636 | 6.30 | 84.933 | 0.74 | 7.1644 | 0.63 | 3.9281 |
| 6.30 | 3.7357 | 5.40 | 81.197 | 0.63 | 2.6277 | 0.54 | 1.3004 |
| 5.40 | 2.8737 | 4.60 | 78.323 | 0.54 | 0.9271 | 0.46 | 0.3733 |
| 4.60 | 1.8486 | 4.00 | 76.475 | 0.46 | 0.2646 | 0.40 | 0.1087 |
| 4.00 | 1.7900 | 3.40 | 74.685 | 0.40 | 0.0895 | 0.34 | 0.0192 |
| 3.40 | 1.4904 | 2.90 | 73.194 | 0.34 | 0.0166 | 0.29 | 0.0025 |
| 2.90 | 1.1886 | 2.50 | 72.006 | 0.29 | 0.0022 | 0.25 | 0.0004 |
| 2.50 | 0.8609 | 2.20 | 71.145 | 0.25 | 0.0003 | 0.22 | 0.0001 |
| 2.20 | 1.4865 | 1.80 | 69.658 | 0.22 | 0.0001 | 0.18 | 0.0000 |
| 1.80 | 1.2945 | 1.60 | 68.364 | 0.18 | 0.0000 | 0.16 | 0.0000 |
| 1.60 | 2.5388 | 1.40 | 65.825 | 0.16 | 0.0000 | 0.14 | 0.0000 |
| 1.40 | 6.5491 | 1.20 | 59.276 | 0.14 | 0.0000 | 0.12 | 0.0000 |
| 1.20 | 16.920 | 1.00 | 42.356 | 0.12 | 0.0000 | 0.10 | 0.0000 |

## Fig. 15 (contd.)

Volume Distribution by Geometric Diameter

Fig. 16

EP 1 409 123 B1

| STATISTICS | | PARARMETERS | | % UNDER | SIZE | % UNDER | SIZE |
|---|---|---|---|---|---|---|---|
| Mean Size | : 1.142 | Material | : Assumed | 5% | 0.6054 | 55% | 1.040 |
| Standard Deviation | : 1.823 | Density | : 1.30 | 10% | 0.6840 | 60% | 1.081 |
| d (4, 3) | : 1.481 | Run Length | : 291.4 | 15% | 0.7390 | 65% | 1.128 |
| D (3, 2) | : 1.003 | Meas. range | : Standard | 20% | 0.7841 | 70% | 1.182 |
| Mode (Linear Scale) | : 0.92 | Sum of Channels | : 352616 | 25% | 0.8239 | 75% | 1.249 |
| Specific Surface Area | : 4.60 sq mtre/g | Lower Size Limit | : 0.10 | 30% | 0.8608 | 80% | 1.341 |
| | | Upper Size Limit | : 200.00 | 35% | 0.8961 | 85% | 1.500 |
| Scans 53 and 54 combined between: | | | | 40% | 0.9309 | 90% | 2.170 |
| 1.6 and 1.6 microns | | | | 45% | 0.9659 | 95% | 6.033 |
| | | | | 50% | 1.002 | | |
| | | Regularization | : High | | | | |

| UPPER SIZE | % IN | LOWER SIZE | % UNDER | UPPER SIZE | % IN | LOWER SIZE | % UNDER |
|---|---|---|---|---|---|---|---|
| | | | | 100 | 0.0000 | 86.0 | 100.00 |
| | | | | 86.0 | 0.0000 | 74.0 | 100.00 |
| | | | | 74.0 | 0.0000 | 63.0 | 100.00 |
| | | | | 63.0 | 0.0000 | 54.0 | 100.00 |
| | | | | 54.0 | 0.0000 | 46.0 | 100.00 |
| | | | | 46.0 | 0.0000 | 40.0 | 100.00 |
| | | | | 40.0 | 0.0000 | 34.0 | 100.00 |
| | | | | 34.0 | 0.0000 | 29.0 | 100.00 |
| | | | | 29.0 | 0.0000 | 25.0 | 100.00 |
| | | | | 25.0 | 0.0000 | 22.0 | 100.00 |
| | | | | 22.0 | 0.0000 | 18.0 | 100.00 |
| 180 | 0.0000 | 160 | 100.00 | 18.0 | 0.0000 | 16.0 | 100.00 |
| 160 | 0.0000 | 140 | 100.00 | 16.0 | 0.0000 | 14.0 | 100.00 |
| 140 | 0.0000 | 120 | 100.00 | 14.0 | 0.0000 | 12.0 | 100.00 |
| 120 | 0.0000 | 100 | 100.00 | 12.0 | 0.0000 | 10.0 | 100.00 |

| UPPER SIZE | % IN | LOWER SIZE | % UNDER | UPPER SIZE | % IN | LOWER SIZE | % UNDER |
|---|---|---|---|---|---|---|---|
| 10.0 | 0.2188 | 8.60 | 99.781 | 1.00 | 19.844 | 0.86 | 29.888 |
| 8.60 | 2.6516 | 7.40 | 97.130 | 0.86 | 14.784 | 0.74 | 15.104 |
| 7.40 | 1.7212 | 6.30 | 95.408 | 0.74 | 8.7965 | 0.63 | 6.3079 |
| 6.30 | 1.3966 | 5.40 | 94.012 | 0.63 | 3.8225 | 0.54 | 2.4854 |
| 5.40 | 1.1490 | 4.60 | 92.863 | 0.54 | 1.6281 | 0.46 | 0.8573 |
| 4.60 | 0.6032 | 4.00 | 92.260 | 0.46 | 0.5529 | 0.40 | 0.3044 |
| 4.00 | 0.5967 | 3.40 | 91.663 | 0.40 | 0.2273 | 0.34 | 0.0772 |
| 3.40 | 0.5426 | 2.90 | 91.120 | 0.34 | 0.0613 | 0.29 | 0.0158 |
| 2.90 | 0.5080 | 2.50 | 90.612 | 0.29 | 0.0131 | 0.25 | 0.0027 |
| 2.50 | 0.5423 | 2.20 | 90.070 | 0.25 | 0.0022 | 0.22 | 0.0005 |
| 2.20 | 1.5044 | 1.80 | 88.566 | 0.22 | 0.0005 | 0.18 | 0.0000 |
| 1.80 | 1.8714 | 1.60 | 86.694 | 0.18 | 0.0000 | 0.16 | 0.0000 |
| 1.60 | 4.4032 | 1.40 | 82.291 | 0.16 | 0.0000 | 0.14 | 0.0000 |
| 1.40 | 10.802 | 1.20 | 71.489 | 0.14 | 0.0000 | 0.12 | 0.0000 |
| 1.20 | 21.757 | 1.00 | 49.732 | 0.12 | 0.0000 | 0.10 | 0.0000 |

## Fig. 16 (contd.)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0322687 A
- WO 9003782 A
- WO 9501221 A
- WO 9600610 A
- WO 9836825 A
- WO 9944733 A
- WO 9959710 A
- WO 0103821 A
- WO 0115664 A
- WO 0238127 A
- WO 9952507 A
- WO 9952550 A
- WO 0030612 A
- WO 0030613 A
- WO 0067892 A
- WO 9731691 A
- US 5770559 A
- WO 0056439 A
- GB 2187115 A
- WO 0166090 A
- GB 0208742 A

### Non-patent literature cited in the description

- **GALLAGHER et al.** Supercritical Fluid Science and Technology. *ACS Symp. Ser.,* 1989, vol. 406, 334
- **RANDOLPH et al.** *Biotechnol. Prog.,* 1993, vol. 9, 429-435
- **ANGUS et al.** International thermodynamic tables of the fluid state - 3. Carbon dioxide. Pergamon Press, 1976